(19) Europäisches Patentamt
European Patent Office
Office européen des brevets

(11) Numéro de publication: **0 255 424 B1**

# (12) FASCICULE DE BREVET EUROPEEN

(45) Date de publication de fascicule du brevet: **18.09.91**

(51) Int. Cl.5: **C07K 15/00**, A61K 39/395

(21) Numéro de dépôt: **87401657.9**

(22) Date de dépôt: **15.07.87**

## (54) Immunotoxines, procédé de préparation et compositions pharmaceutiques en contenant.

(30) Priorité: **15.07.86 FR 8610297**

(43) Date de publication de la demande:
**03.02.88 Bulletin 88/05**

(45) Mention de la délivrance du brevet:
**18.09.91 Bulletin 91/38**

(84) Etats contractants désignés:
**AT BE CH DE ES FR GB GR IT LI LU NL SE**

(56) Documents cités:
**EP-A- 0 074 279**
**EP-A- 0 169 111**
**FR-A- 2 312 259**

**CHEMICAL ABSTRACTS, vol. 96, no. 11, 15 mars 1982, page 40, résumé no. 79573c, Columbus, Ohio, US; Y. XIONG et al.: "Effect of trichosanthin on different kinds of cultured human cells in vitro", & SHIYEN SHENGWU XUEBAO 1981, 14(3), 259-69**

**CHEMICAL ABSTRACTS, vol. 95, no. 4, 27 juillet 1981, page 328, résumé no. 30276y, Columbus, Ohio, US; SHANGHAI INSTITUTE OF ORGANIC CHEMISTRY: "Chemistry of trichosanthin", & NUCLEIC ACIDS PROTEINS, PROC. SYMP. 1979 (Pub. 1980), 318-23**

(73) Titulaire: **SANOFI S.A.**
**40, Avenue George V**
**F-75008 Paris(FR)**

(72) Inventeur: **Barbieri, Luigi**
**Via San Giorgio 2616 B**
**I-40024 Castel San Pietro Terme Bologna(IT)**
Inventeur: **Casellas, Pierre**
**Rue de l'Alguelongue**
**La Colombe No. 9 F-34100 Montpellier(FR)**
Inventeur: **Stirpe, Florenzo**
**Via San Petronio Vecchio**
**I-40125 Bologna(IT)**

(74) Mandataire: **Gillard, Marie-Louise**
**Cabinet Beau de Loménie 55, Rue d'Amsterdam**
**F-75008 Paris(FR)**

**EP 0 255 424 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet des produits, appartenant à la classe des immunotoxines, obtenus par couplage covalent entre, d'une part la trichokirine telle quelle ou convenablement modifiée (ci-après désignée par le symbole A) et, d'autre part un anticorps ou fragment d'anticorps, utilisé sous sa forme naturelle ou correctement modifié (ci-après désigné par le symbole P), possédant la capacité de reconnaître sélectivement un antigène porté par les cellules à détruire visées. Le couplage entre les 2 protéines peut être réalisé soit par l'intermédiaire d'une liaison disulfure, soit par l'intermédiaire d'une liaison thioéther.

Ainsi, la présente invention a pour objet, selon un de ses aspects, une immunotoxine formée par couplage d'un anticorps P avec la trichokirine, protéine extraite de la plante Trichosanthes kirilowii, ayant la formule statistique suivante :

P' - W - A'    (I)

dans laquelle
P' représente le radical d'une protéine qui est un anticorps ou un fragment d'anticorps tel quel ou chimiquement convenablement modifié, privée d'au moins l'une de ses fonctions propres et dans lequel les autres groupes fonctionnels sont éventuellement bloqués. A' représente le radical d'une protéine qui est la trichokirine telle quelle ou chimiquement convenablement modifiée, privée d'au moins une de ses fonctions propres et dans laquelle les autres groupes fonctionnels sont éventuellement bloqués et W représente une structure covalente bivalente contenant au moins un groupe thioéther ou un groupe disulfure dont l'un des atomes de sulfure est soit choisi parmi ceux des cystéines de P ou A, soit lié aux fonctions propres de P et/ou de A par des structures d'espacement portant un groupe fonctionnel lié auxdites fonctions propres de P et/ou A.

Par liaison thioéther entre deux protéines, on entend une liaison du type :

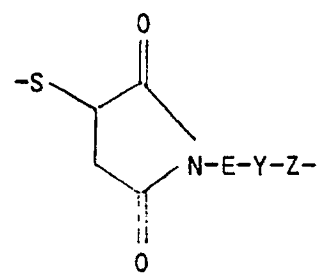

dans laquelle
Z, Y et E sont tels que définis ci-dessous.

D'une façon préférentielle, la présente invention concerne une immunotoxine de formule statistique :

P' - W' - A'    (II)

dans laquelle
P' et A' sont tels que définis ci-dessus et W' représente une structure covalente choisie parmi :

(a) un groupement de formule :

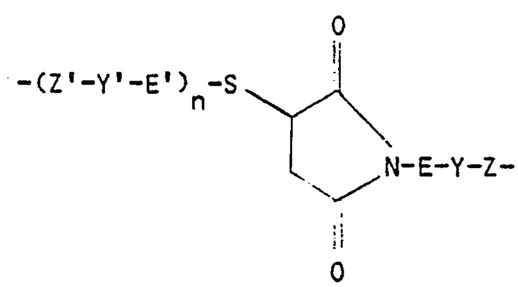

(b) un groupement de formule :

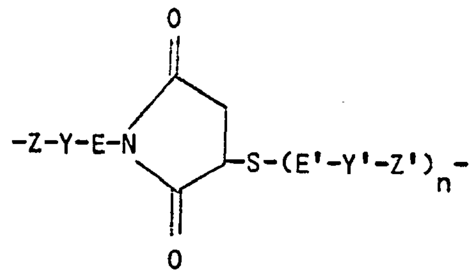

(c) un groupement de formule :

-Z'-Y'-E'-S-S-(E-Y-Z)$_n$-

(d) un groupement de formule :

-S-S-(E-Y-Z)$_n$-

où

- Z et Z' identiques ou différents représentent les fonctions propres des protéines A et P, choisies parmi l'atome d'oxygène provenant de l'hydroxyle de l'un des résidus de tyrosine ; le groupe carbonyle provenant de l'un des carboxyles terminaux ou des carboxyles libres des acides aspartiques et/ou glutamiques de A et de P ; le groupe provenant de la structure dialdéhydique obtenue après oxydation de la structure glucidique de P ou le cas échéant de la protéine A par l'acide periodique ; et le groupe -NH- provenant de l'une des amines terminales de A et de P ou de l'une des amines en position epsilon de l'un des résidus de lysine ;
- Y et Y' représentent des groupes engagés dans une liaison covalente avec l'une quelconque des fonctions Z et Z' des protéines A et P ;
- E et E' représentent des structures d'espacement inertes ;
- n représente zéro ou 1.

Les immunotoxines de la présente invention sont représentées par les formules (I) et (II) ci-dessus en forme simplifiée, mais il est entendu qu'il peut y avoir plusieurs structures W ou W' liées à une même molécule de P et/ou de A, et donc plusieurs A liés à un seul P et réciproquement ; le nombre de ponts dépendant du procédé de couplage et du nombre de fonctions propres de P et de A. Ainsi, les formules statistiques I et II représentent aussi ces produits et leurs mélanges, qui répondent à la formule P' (W'-A')m, dans laquelle m est un nombre entier ou fractionnaire, inférieur ou supérieur à 1.

Par exemple, si une immunotoxine est formée par couplage de la trichokirine avec l'anticorps P (par exemple l'anticorps T101) par l'intermédiaire d'une structure covalente bivalente ayant un groupe disulfure dont un soufre est celui d'une cystéine de la trichokirine et l'autre est lié aux oxygènes phénoliques des tyrosines de l'anticorps P par un groupe oxopropylique, elle aura la formule statistique :

P'(O-CO-$CH_2$-$CH_2$-S-S-A')$_t$

dans laquelle
t représente le nombre des tyrosines de l'anticorps (par exemple de l'anticorps T101) intervenues dans le couplage.

L'immunotoxine ainsi obtenue correspond à un produit de formule (II), où :

- P' est tel que défini ci-dessus, notamment le radical de l'anticorps T101 privé de t fonctions phénoliques de ses tyrosines ;
- A' est tel que défini ci-dessus, notamment le radical de la trichokirine privée de la fonction thiol d'une de ses cystéines ;
- W' est le groupement (c) : -Z'-Y'-E'-S-S-(E-Y-Z)$_n$- où Z' est l'oxygène des hydroxyles phénoliques intervenus dans le couplage, Y est -CO-, E est la structure d'espacement inerte -$CH_2$-$CH_2$- et n est zéro.

Particulièrement préférées sont les immunotoxines formées par une ou plusieurs structures contenant la trichosanthine ou la trichokirine et un anticorps P, représentées par la formule statistique :

$P'(W' - A')_m$ (III)

dans laquelle
P', W' et A' sont tels que définis ci-dessus et m représente le nombre de fonctions propres de la protéine P intervenues dans le couplage. Le nombre m varie de 0,3 à 12, de préférence de 0,5 à 10.

L'expression "m varie de 0,3 à 12, de préférence de 0,5 à 10" signifie que la valeur de m est statistique car dans la population des molécules d'anticorps, le couplage ne se produit pas d'une façon homogène. Le nombre m peut donc ne pas être entier.

La valeur de m dépend notamment des anticorps utilisés, plus particulièrement de leur poids moléculaire.

Ainsi, si comme anticorps P de départ on utilise un fragment Fab ou Fab', la valeur de m pourra varier entre 0,3 et environ 2 ; si l'on utilise un fragment $F(ab')_2$, m pourra varier entre 0,5 et environ 4 ; pour un anticorps du type IgG, la valeur de m sera entre 0,5 et 6 ; enfin, pour un anticorps IgM, la valeur de m pourra varier entre 1 et 12.

Il est cependant préférable que le degré de substitution sur l'anticorps P soit tel qu'il conduise à une valeur de m non inférieure à 0,5 et non supérieure à 10.

Plus généralement, les structures (I) et (II) ci-dessus représentent des formules statistiques écrites de façon simplifiée, comme il a été spécifié ci-dessus.

D'une façon analogue, les formules (IV), (V) et (VI) ci-dessous sont également statistiques - lorsque, le cas échéant, n est 1 - car les réactifs de couplage sont préparés à partir de populations de protéines P1 et P2 qui ont toutes exactement les mêmes propriétés que celles prises en compte ci-dessus pour l'anticorps P, que ces protéines P1 et P2 soient elles-mêmes l'anticorps P ou la protéine extraite de Trichosanthes kirilowii.

Les immunotoxines de formule statistique

$$P'(NH\text{-}CO\text{-}CH_2CH_2\text{-}S\text{-}S\text{-}\underset{\displaystyle CH_2COOH}{\underset{|}{CH}}\text{-}CO\text{-}NH\text{-}TK')_m \quad \text{(IIIa)}$$

dans laquelle
P' et m sont tels que définis ci-dessus et TK' est le radical de la trichokirine telle quelle, privée de fonctions amine, et les immunotoxines de formule statistique

$P'(NH\text{-}CO\text{-}CH_2CH_2\text{-}S\text{-}S\text{-}CH_2CH_2\text{-}CO\text{-}O\text{-}TK'')_m$ (IIIb)

dans laquelle
P' et m sont tels que définis ci-dessus et TK" est le radical de la trichokirine telle quelle, privée de fonctions hydroxyle phénolique,
sont particulièrement actives.

Selon un autre de ses aspects, la présente invention concerne un procédé pour la préparation d'une immunotoxine ayant la structure (I) ci-dessus, caractérisé en ce qu'on fait réagir en solution aqueuse et à la température ambiante une protéine P1, qui est indifféremment soit la trichokirine éventuellement modifiée, soit un anticorps ou fragment d'anticorps, porteuse d'au moins un groupe thiol libre attaché à ladite protéine P1 directement ou par l'intermédiaire d'une structure d'espacement, avec une protéine P2, différente de P1, qui est indifféremment soit la trichokirine éventuellement modifiée, soit un anticorps ou fragment d'anticorps, porteuse d'un groupement capable de couplage avec le thiol libre de la protéine P1, de façon à former une liaison thioéther ou disulfure.

Selon un aspect préférentiel, la présente invention concerne un procédé pour la préparation d'une immunotoxine ayant la structure (II), dans laquelle P', W' et A' sont tels que définis ci-dessus, caractérisé en ce qu'on fait réagir en solution aqueuse et à la température ambiante une protéine de formule ::

$P1'\text{-}(Z\text{-}Y\text{-}E)_n\text{-}SH$ (IV)

avec une protéine de formule statistique :

P2'-Z'-Y'-E'-G (V)

où P1' et P2' représentent les radicaux des protéines P1 et P2 liés aux fonctions propres desdites protéines ou les radicaux de l'une des protéines P1 ou P2 provenant de l'ouverture des structures glucidiques des anticorps ou fragments d'anticorps ou le cas échéant de la protéine A par action de l'acide periodique, Z,Z', Y,Y', E et E' sont tels que définis ci-dessus et G représente un groupe :

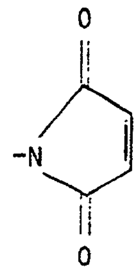

ou un groupe -S-S-X, où X est un groupe activateur.

Autant P que A sont donc des protéines qui présentent indifféremment :

(1) la ou les fonctions thiol qui participent au couplage ;

(2) un ou plusieurs groupements fonctionnels capables de réagir avec les fonctions thiol ci-dessus pour former une liaison disulfure ou thioéther.

Selon la présente invention, lesdites fonctions thiol et groupements fonctionnels sont ceux des protéines P ou A natives, ou bien ils y sont introduits artificiellement.

Pour des raisons de clarté, on précise ci-après la signification des symboles utilisés pour désigner les protéines ci-dessus ou leurs radicaux et des expressions utilisées pour désigner les différents symboles.

Le symbole P représente une protéine choisie parmi tout anticorps ou fragment d'anticorps ou toute immunoglobuline ou fragment d'immunoglobuline ou toute molécule dérivant des précédentes par modification artificielle de l'un quelconque de leurs groupements fonctionnels, y compris des structures glucidiques qu'elles portent, sous réserve que la protéine ainsi choisie reste capable de reconnaître sélectivement un antigène donné à la surface des cellules porteuses de cet antigène et notamment des cellules cancéreuses.

Le symbole A représente une protéine qui est la trichokirine telle qu'elle peut être directement obtenue à partir de Trichosanthes kirilowii ou toute molécule dérivant de cette protéine par modification artificielle de tout groupement fonctionnel porté par cette même protéine sous réserve que la protéine ainsi choisie présente encore la propriété d'inhiber la synthèse protéique ribosomale des cellules eucaryotes, telle que l'on peut la mettre en évidence dans un modèle d'étude acellulaire.

Le symbole P' représente un radical dérivé de la protéine P ci-dessus, telle quelle ou chimiquement convenablement modifiée, privée d'une ou plusieurs fonctions propres et dont d'autres groupes fonctionnels sont éventuellement bloqués.

Le symbole A' représente un radical dérivé de la protéine A ci-dessus, telle quelle ou chimiquement convenablement modifiée, privée d'une ou plusieurs fonctions propres et dont d'autres groupes fonctionnels sont éventuellement bloqués.

Le symbole P1 représente une des protéines A et P telles que définies ci-dessus qui porte des fonctions thiol libres, attachées à ladite protéine directement ou par l'intermédiaire d'une structure d'espacement.

Le symbole P2, différent de P1, représente une des protéines A et P telles que définies ci-dessus, qui porte un ou plusieurs groupements fonctionnels capables de réagir avec les thiols libres.

Le symbole P1' représente le radical de la protéine P1 qui porte les fonctions propres de la protéine P1 notamment les fonctions SH (de la cystéine), $NH_2$ (terminal de la protéine ou dans la position epsilon des lysines), OH (des tyrosines), COOH (des acides aspartiques et glutamiques) ou le radical de la protéine P1, provenant de l'ouverture des structures glucidiques par action de l'acide periodique lorsque P1 désigne un anticorps ou fragment d'anticorps, ou le cas échéant la protéine A.

Le symbole P2' représente le radical de la protéine P2 qui porte les groupes fonctionnels caractéristiques $NH_2$ (terminal de la protéine ou dans la position epsilon des lysines), OH (des tyrosines), COOH (des acides aspartiques et glutamiques).

Par exemple, P1'-SH représente la protéine P1 (qui peut être indifféremment l'anticorps ou fragment d'anticorps P ou la trichokirine) dans laquelle les groupes SH des cystéines sont libres et les autres

groupes fonctionnels sont éventuellement bloqués.

De la même façon, P1'-CO- représente la protéine P1, dans laquelle son groupe carboxylique terminal ou les groupes carboxyliques de ses acides glutamiques et aspartiques sont couplés avec un groupement qui apporte un groupe SH introduit artificiellement.

Encore, P2'-NH- représente la protéine P2 (qui peut être indifféremment l'anticorps ou fragment d'anticorps P ou la trichokirine) dans laquelle son groupe amino terminal ou les groupes amino de ses lysines sont attachés à un groupement susceptible de couplage avec le thiol de la protéine P1.

Le terme "structure d'espacement inerte" tel qu'utilisé ici pour E et E' désigne un radical organique bivalent inerte vis-à-vis des réactifs utilisés dans le procédé, tel qu'un groupe alkylène à chaîne droite ou ramifiée contenant de 1 à 15 atomes de carbone, qui peut contenir une ou plusieurs doubles liaisons ou qui peut être interrompu par des atomes d'oxygène ou qui peut être substitué par un ou plusieurs groupes fonctionnels inertes, tels que des groupes méthoxy, des groupes carboxyles libres ou estérifiés, des groupes dialkylamino, des groupes carbamates. Le même terme désigne également un groupe arylène contenant de 6 à 15 atomes de carbone qui peut être substitué par un ou plusieurs groupes fonctionnels inertes comme indiqué ci-dessus pour le groupe alkylène.

L'expression "groupe fonctionnel capable de se lier d'une façon covalente" telle qu'utilisée ici pour Y et Y' désigne tous les groupes susceptibles de réagir avec les fonctions propres des protéines P1 et P2 pour donner une liaison covalente. Ainsi, par exemple, les groupes -CO- sont des groupes fonctionnels convenables susceptibles de se lier aux amines libres, aux thiols et aux hydroxyles phénoliques des protéines. De même, le groupe -NH- est un groupe fonctionnel convenable susceptible de se lier aux groupes carboxyliques libres des protéines. Le groupe =N- est un groupe fonctionnel convenable, susceptible de se lier aux deux atomes de carbone des structures glucidiques des protéines P1 et P2 après oxydation avec les ions périodate lorsque P1 ou P2 représente un anticorps ou fragment d'anticorps ou, le cas échéant, la protéine A.

L'expression "fonction propre des protéines", telle qu'utilisée ici pour Z, et Z' désigne les radicaux provenant des groupes caractéristiques des acides aminés formant les protéines P1 et P2, telles que l'atome d'oxygène provenant des hydroxyles des acides aminés tyrosine et éventuellement sérine, le groupe carbonyle provenant du carboxyle terminal ou des carboxyles libres des acides aspartique et glutamique, le groupe -NH- provenant de l'amine terminale des protéines ou des lysines, l'atome de soufre provenant du thiol de la cystéine. La même expression désigne également le groupe provenant de la structure dialdéhydique obtenue après oxydation d'une des structures glucidiques des protéines P1 ou P2 par traitement avec les ions périodate lorsque P1 ou P2 représente un anticorps ou fragment d'anticorps, ou le cas échéant la protéine A.

Le terme "radical activateur", tel qu'utilisé ici pour X, désigne un groupement lié à un pont -S-S- capable de réagir avec un thiol libre pour former un disulfure avec libération de X-SH. Des radicaux activateurs convenables sont les groupes pyridyl-2 et pyridyl-4, non substitués ou substitués par un ou des halogènes ou des radicaux alkyle, carboxyle, alcoxycarbonyle ; le groupe phényle non substitué ou, de préférence, substitué par un ou des halogènes ou des groupes nitro, alcoxy, carboxyle ou alcoxycarbonyle ; ou un groupe alcoxycarbonyle tel que méthoxycarbonyle.

Les termes "alkyle" et "alcoxy" désignent des groupes contenant jusqu'à 5 atomes de carbone.

Le terme "alkylène" désigne des groupements aliphatiques saturés, à chaîne droite ou ramifiée contenant jusqu'à 10 atomes de carbone pouvant être substitués par un ou plusieurs groupes fonctionnels inertes tels que les groupes alcoxycarbonyle.

Par extraction des graines de Trichosanthes kirilowii, on obtient une nouvelle proteine inactivant les ribosomes (RIP) exceptionnellement puissante, différente de la trichosanthine. Cette nouvelle RIP, nommée "trichokirine", a les caractéristiques suivantes :

- Glycoprotéine ayant un poids moléculaire 28 000 ± 3 000, déterminé par électrophorèse sur gel de polyacrylamide en présence de SDS (sodium dodécyl sulfate),
- de point isoélectrique ≧ 9
- de contenu en sucres neutres : 1,1 à 1,5 % en poids dont 0,3 à 1,2 % de mannose,
- de composition en acides aminés : nombre de résidus par mole de protéine ± 20 % :

| | | | | | | | | | | |
|---|---|---|---|---|---|---|---|---|---|---|
| Lys : 17,3 | ; | His : 1,1 | ; | Arg | : 6,7 | ; | Asx : 22,8 | ; |
| Thr : 18,9 | ; | Ser : 23,5 | ; | Glx | : 21,6 | ; | Pro : 8,0 | ; |
| Gly : 16,0 | ; | Ala : 21,1 | ; | 1/2 Cys | : 1,9 | ; | | |
| Val : 12,5 | ; | Met : 3,05 | ; | Ile | : 15,8 | ; | Leu : 24,3 | ; |
| Tyr : 12,1 | ; | Phe : 10,1 | ; | Trp | : n.d.. | | | |

tel que Asx est l'ensemble des résidus d'acide aspartique et d'asparagine

Glx est l'ensemble des résidus d'acide glutamique et de glutamine

1/2 Cys est le résidu de cystéine de la protéine native sous la forme d'acide cystéique déterminé lors de l'analyse,

et n.d. est non déterminé.

- de séquence amino-terminale :

1 10

Asp-Val-Ser-Phe-Ser-Leu-Ser-Gly-Gly-Gly-

16

Thr-Ala-Ser-Tyr-Glu-Lys

La composition en acides aminés est déterminée par chauffage à 105° C pendant 24 heures sous azote en présence d'acide chlorhydrique 6 N, phénol à 1 % et 2-mercaptoéthanol à 1 %. Les acides aminés sont dérivés par le phénylisothiocyanate (PITC) en milieu alcalin. Les phénylthiohydantoïnes (PTH) ainsi obtenues sont analysées par HPLC sur phase inverse. La cystéine a été déterminée après oxydation de la protéine avec l'acide performique (J. Biol. Chem. 238, 235-237 (1963)).

La séquence amino-terminale est déterminée avec un microséquenceur commercialisé par Applied Biosynthesis, à partir de 5 nmoles de protéine solubilisées dans une solution d'acide acétique à 10 %. Les PTH des aminoacides sont analysées par HPLC sur phase inverse.

La trichokirine est obtenue par un procédé caractérisé en ce que l'on extrait des graines broyées de Trichosanthes kirilowii avec de l'eau en présence d'un tampon à pH 6,5-7,5, on élimine le matériau insoluble, on fractionne l'extrait brut par chromatographie sur une résine échangeuse d'ions faiblement acide, en éluant avec une solution tampon contenant du chlorure de sodium à un pH de 7,2-7,7 et on isole la fraction contenant la protéine inhibitrice et la purifie.

Par exemple, les graines de T. kirilowii, de préférence après décorticage, sont broyées et extraites avec de l'eau contenant un électrolyte, par exemple du chlorure de sodium, et un tampon, par exemple un tampon phosphate, à un pH voisin de la neutralité. Les matières insolubles sont éliminées par exemple par centrifugation et les substances de bas poids moléculaire sont éliminées de la solution par dialyse par exemple contre du tampon phosphate. L'extrait dialysé est alors soumis à un fractionnement, par exemple par chromatographie sur une résine échangeuse d'ions faiblement acide telle que la carboxyméthylcellulose. L'élution peut être effectuée en utilisant un gradient linéaire de concentration par exemple une solution aqueuse de 0 à 0,3 M de chlorure de sodium. Normalement, 4 pics principaux sont élués et le pic IV élué par une concentration en chlorure de sodium de 0,03 à 0,11 M contient la plus grande partie de la trichokirine.

La purification de la trichokirine peut être effectuée par filtration sur gel par exemple sur une colonne de Sephadex (marque déposée).

La trichokirine peut être couplée aux anticorps ou fragments d'anticorps par l'intermédiaire d'un agent de couplage approprié selon le procédé décrit ci-dessus.

La trichokirine inhibe les synthèses protéiques dans un lysat de réticulocytes avec une $DI_{50}$ de 1,5-4 nano.g/ml. Sur les cellules intactes, l'effet est de loin inférieur, avec une $DI_{50}$ qui peut varier de 7 à plus de 100 micro.g/ml, selon la cellule examinée.

Le tableau 1 montre l'effet de la trichokirine sur diverses cellules, notamment la $DI_{50}$ (micro.g/ml et en $M.10^{-7}$) de l'inhibition de la synthèse protéique ; celle-ci a été déterminée en appliquant la méthode décrite dans J. Biol. Chem. 259, 9359-9354 (1984).

TABLEAU 1

| Cellule | : $DI_{50}$ micro.g/ml | : $M.10^{-7}$ |
|---|---|---|
| Fibroblastes humains | : 3,9 | : 1,3 |
| Cellules TG | : 7,5 | : 2,5 |
| Cellules NB 100 (neuroblastome) | : 10,3 | : 3,4 |
| Cellules JAR (choriocarcinome) | : 16,3 | : 5,4 |
| Cellules HeLa | : 45,0 | : 15,0 |
| M 4039 (melanome) | : 9,9 | : 3,3 |

La préparation d'anticorps monoclonaux dirigés contre des cellules cancéreuses humaines a été largement mentionnée dans la littérature scientifique et beaucoup de ces anticorps sont maintenant disponibles commercialement.

Le couplage chimique entre la trichokirine et l'anticorps (ou fragment d'anticorps) peut être réalisé selon le procédé de la présente invention par des modes opératoires qui :

- préservent les activités biologiques respectives des deux composants du conjugué : l'anticorps et la trichosanthine ou la trichokirine,
- assurent au procédé une reproductibilité satisfaisante et un bon rendement de couplage,
- permettent de maîtriser la valeur du rapport toxine/anticorps dans le conjugué obtenu,
- conduisent à un produit stable et soluble dans l'eau.

Parmi les modes opératoires répondant à ces caractéristiques, il faut privilégier ceux qui mettent en oeuvre une ou plusieurs fonctions thiol pour l'établissement de la liaison entre les 2 protéines. En effet, ces fonctions thiol se prêtent particulièrement bien à l'établissement, soit de liaisons disulfure, soit de liaisons thioéther qui satisfont les unes comme les autres aux conditions générales ci-dessus. Ces modes opératoires sont décrits dans le brevet US 4 340 535 et le brevet EP 169 111.

D'une façon générale, pour la bonne conduite des réactions de couplage entre protéines et en vue d'éliminer notamment les réticulations anarchiques, il importe que l'une des protéines à coupler et elle seule porte la ou les fonctions thiol à mettre en oeuvre, alors que l'autre protéine et elle seule porte une ou plusieurs fonctions susceptibles de réagir avec les thiols en milieu aqueux de pH compris entre 5 et 9 et à une température ne dépassant pas 30 °C, pour fournir une liaison covalente stable et définie.

Les caractéristiques des protéines P1 et P2 utilisées comme produits de départ sont illustrées en détail ci-après. La structure d'espacement E peut être remplacée par les structures préférentielles R à R8 qui ne sont données qu'à titre d'exemple.

CAS DE LA PROTEINE P1

Cette protéine étant dans tous les cas celle qui porte la ou les fonctions thiol qui participeront au couplage, la situation se présente de façon diverse selon la nature de cette protéine P1.

A) La protéine P1 porte naturellement un ou plusieurs radicaux thiol utilisables pour permettre le couplage à la protéine P2 ; c'est notamment le cas si la protéine P1 est le fragment d'anticorps connu sous la dénomination de F(ab'), tel qu'il est classiquement obtenu par protéolyse limitée de l'anticorps en présence de pepsine, suivie d'une réduction du (ou des) ponts disulfure entre chaînes lourdes.

C'est également le cas si la protéine P1 est la trichokirine ou un dérivé de cette toxine où l'un au moins des groupements thiol portés par les résidus cystéine de la toxine native est libre et accessible au couplage

chimique.

Dans tous les cas, la protéine P1 porteuse de son (ou de ses) groupement(s) thiol naturel(s) peut être utilisée dans cet état pour l'étape de couplage.

B) La protéine P1 ne porte pas naturellement de radicaux thiol utilisables pour permettre le couplage à la protéine P2 :

- c'est notamment le cas si la protéine P1 est une immunoglobuline native, un anticorps entier ou un fragment d'anticorps et notamment l'un des fragments couramment dénommés F(ab)'2 ou F(ab) ;
- un autre cas où la protéine P1 ne porte pas naturellement de fonction thiol utilisable pour le couplage est celui où cette protéine P1 est la trichokirine où chacun des résidus de cystéine est soit bloqué par alkylation, soit inaccessible à la modification chimique.

Dans tous les cas, il conviendra alors d'introduire artificiellement sur de telles molécules une ou plusieurs fonctions thiol aptes à permettre le couplage.

Trois types de réactions peuvent être utilisés de préférence pour l'introduction de fonctions thiol :

1 - Le premier type de réaction est l'action de l'anhydride S-acétylmercaptosuccinique qui est capable d'acyler des fonctions aminées de la protéine. On pourra ensuite libérer les fonctions thiol par élimination du radical acétyl protecteur, par action de l'hydroxylamine, ainsi que cela a été décrit (Archives of Biochemistry and Biophysics, 119, 41-49, (1967)). On pourra même, dans le cas où la (ou les) fonction(s) thiol ainsi introduite(s) sous forme protégée doivent réagir ultérieurement avec un radical disulfure mixte activé, se dispenser de la déprotection préalable par l'hydroxylamine ; en effet, la réaction de formation de la liaison disulfure utilisant les réactifs, objets de la présente invention, se produit aussi bien avec le radical S-acétyl qu'avec le thiol libre.

D'autres méthodes décrites dans la littérature scientifique peuvent aussi être utilisées pour l'introduction de fonctions thiol sur la protéine à modifier.

Une protéine P1 modifiée particulièrement avantageuse est une S-acétylmercaptosuccinoyl-trichokirine obtenue par traitement de la trichokirine avec l'anhydride S-acétylmercaptosuccinique, ainsi que le dérivé mercaptosuccinoyle correspondant préparé par action de l'hydroxylamine sur le dérivé S-acétyle.

Cette trichokirine modifiée peut être représentée par la formule :

$$TK^{o} - (CO - \underset{CH_2COOH}{CH} - S - Q)_p$$

dans laquelle

$TK^o$ représente le radical de la trichokirine, Q représente l'hydrogène ou le groupe acétyle et p peut varier entre 0,2 et 3, de préférence entre 0,4 et 1.

2 - Le deuxième type de réaction consiste à faire réagir la protéine par ses groupements carboxyliques avec une molécule diaminée symétrique présentant un pont disulfure, de formule :

$H_2N\text{-}R_1\text{-}S\text{-}S\text{-}R_1\text{-}NH_2$

dans laquelle

$R_1$ est un groupe aliphatique comportant de 2 à 5 atomes de carbone.

La réaction se fait de préférence avec la cystamine [$R_1$ = $-(CH_2)_2-$] en présence d'un agent de couplage, tel qu'un carbodiimide et notamment un dérivé soluble dans l'eau comme l'éthyl-1 (diméthylamino-3 propyl)-3 carbodiimide, et conduit à la formation, selon les stoechiométries mises en oeuvre, de l'un des dérivés suivants ou du mélange des deux :

$P1'\text{-}CO\text{-}NH\text{-}R_1\text{-}S\text{-}S\text{-}R_1\text{-}NH_2$ (Ia)

$P1'\text{-}CO\text{-}NH\text{-}R_1\text{-}S\text{-}S\text{-}R_1\text{-}NH\text{-}CO\text{-}P1'$ (Ib).

Un tel produit de réaction peut être alors utilisé de deux manières :

a) Si dans les formules Ia et Ib, le radical P1' est le radical de la protéine P1 qui est la trichokirine ou l'un de ses dérivés, le milieu réactionnel obtenu est soumis sans fractionnement à l'action d'un réducteur, tel que le mercapto-2 éthanol, ce qui conduit à l'obtention d'un dérivé protéique unique de formule générale :

P1'-CONH-$R_1$-SH

Le produit ainsi obtenu est alors purifié par dialyse ou filtration sur gel.

b) Si dans les formules Ia et Ib, le radical P1' est le radical de la protéine P1 constituée par un anticorps ou l'un de ses fragments, le milieu réactionnel obtenu sera utilisé tel quel pour le couplage en utilisant alors une méthode d'échange thiol/disulfure, par exemple celle décrite par Gilliland et Collier (Cancer Research, 40, 3564, (1980)).

3 - Le troisième type de réaction consiste à utiliser des motifs glucidiques naturellement présents sur les anticorps pour fixer le radical porteur du thiol que l'on se propose d'introduire. La protéine est alors soumise à une oxydation par les ions periodate afin de faire apparaître des fonctions aldéhyde sur les motifs glucidiques. La réaction est arrêtée par addition d'un réactif qui consomme le periodate restant, par exemple un excès d'éthylèneglycol et les sous-produits sont éliminés par dialyse ou tout autre traitement approprié. Le produit obtenu est traité par une molécule diaminée symétrique présentant un pont disulfure, de formule générale

$H_2N$-$R_1$-S-S-$R_1$-$NH_2$

dans laquelle

$R_1$ est un groupe aliphatique comportant de 2 à 5 atomes de carbone. Les produits d'addition formés sont alors réduits en amines secondaires ou tertiaires par action d'un hydrure métallique convenable, notamment le borohydrure de sodium. La réaction se fait de préférence avec la cystamine [$R_1$ = -$(CH_2)_2$-] et conduit à la formation, selon les stoechiométries mises en oeuvre de l'un des dérivés suivants ou du mélange des deux :

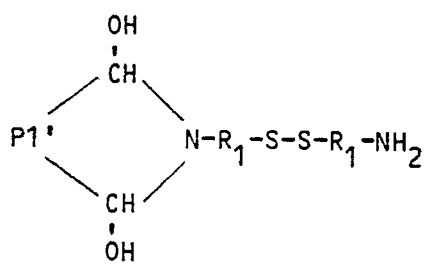

IIa

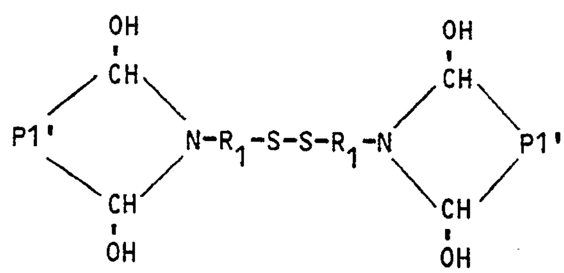

IIb

Le milieu réactionnel pourra alors être traité exactement comme indiqué ci-dessus pour les produits caractérisés par les structures Ia ou Ib, où P1' représente un anticorps ou fragment d'anticorps.

Dans les deux derniers types de réaction d'introduction artificielle de groupements thiols décrits ci-dessus (ceux utilisant un réactif disulfure diaminé symétrique), il est préférable que la protéine P1 mise en oeuvre ne possède pas de groupes SH libres.

Dans le cas des anticorps ou fragments d'anticorps et plus généralement de toutes les substances du premier groupe tel que défini précédemment qui ne possèdent pas de groupements SH naturellement libres, il conviendra de procéder à une méthylation réductive, par exemple selon la méthode de MEANS et FEENEY : on peut ainsi habituellement introduire plusieurs dizaines de radicaux méthyle par mole d'anticorps sans modifier sa capacité à reconnaître sélectivement un antigène à la surface des cellules porteuses de cet antigène.

CAS DE LA PROTEINE P2

Cette protéine est dans tous les cas celle qui porte un ou plusieurs groupements fonctionnels capables de réagir avec les thiols de la protéine P1 pour former une liaison soit disulfure, soit thioéther. Ces groupements fonctionnels, toujours artificiellement introduits sur la protéine P2, diffèrent selon que l'on cherche à obtenir un couplage par liaison disulfure ou par liaison thioéther et sont choisis comme indiqués ci-après.

A) Cas de la liaison disulfure

La préparation du conjugué peut alors être représentée par le schéma :

$P1'-(Z-Y-E)_n-SH + P2'-Z'-Y'-E'-S-S-X \rightarrow$
$P1'-(Z-Y-E)_n-S-S-E'-Y'-Z-'P2' + X-SH$

La protéine P2' substituée par un atome de soufre activé est obtenue à partir de la protéine P2 elle-même ou de la protéine P2 correctement protégée, par substitution à l'aide d'un réactif lui-même porteur d'un atome de soufre activé selon le schéma :

$P2 + L-Y'-R-S-S-X \rightarrow P2'-Z'-Y'-R-S-S-X$

dans lequel :

- P2 désigne la protéine à substituer ;
- L-Y' représente une fonction permettant la fixation covalente du réactif sur la protéine.

Le groupement fonctionnel L-Y' est une fonction capable de se lier de façon covalente avec l'une quelconque des fonctions portées par les chaînes latérales des aminoacides constitutifs de la protéine à substituer. Parmi celles-ci, on retiendra particulièrement :

a) les fonctions aminées terminales des chaînes peptidiques ou les fonctions aminées latérales des radicaux lysyle contenus dans la protéine.

Dans ce cas, L-Y' pourra notamment représenter :

- un groupe carboxylique qui pourra se lier aux fonctions aminées de la protéine en présence d'un agent de couplage tel qu'un carbodiimide et notamment un dérivé soluble dans l'eau comme l'éthyl-1 (diméthylamino-3 propyl)-3 carbodiimide ;
- un chlorure d'acide carboxylique qui est susceptible de réagir directement avec les fonctions aminées pour les acyler ;
- un ester dit "activé" tel qu'un ester d'ortho- ou para-, nitro- ou dinitro-phényle ou encore un ester de N-hydroxy succinimide qui peut réagir directement avec les fonctions aminées pour les acyler ;
- un anhydride interne d'un diacide carboxylique tel, par exemple, l'anhydride succinique qui réagit spontanément avec les fonctions amines pour créer des liaisons amides ;
- un groupement imidoester,

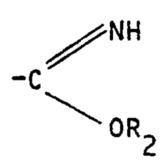

$(L = OR_2, Y' = -(C{=}NH)-)$

où $R_2$ est un groupe alkyle, qui réagit avec les groupes aminés de la protéine P2 selon la réaction :

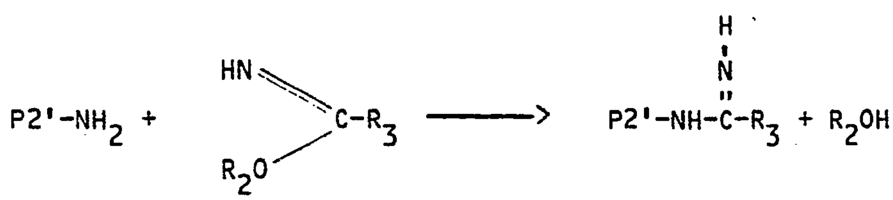

où $R_3$ représente le groupe -R-S-SX ;

b) les fonctions phénol des radicaux tyrosyle contenus dans la protéine.

Dans ce cas, L-Y' pourra notamment représenter un groupement imidazolyl-1 carbonyl, réagissant avec les groupes phénol de la protéine selon la réaction :

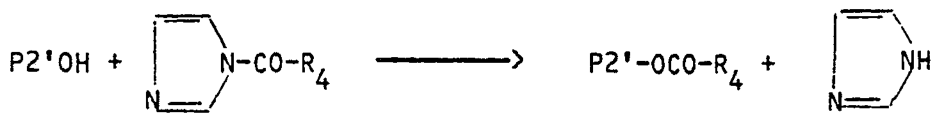

où le 1-imidazolyle est L, le groupe CO est Y' et $R_4$ est le groupe -R-S-S-X.

Le radical -S-S-X désigne un disulfure mixte activé capable de réagir avec un radical thiol libre. En particulier, dans ce disulfure mixte, X pourra désigner un groupe pyridyl-2 ou pyridyl-4, éventuellement substitué par un ou des radicaux alkyle, halogène ou carboxylique. X peut aussi désigner un groupe phényle de préférence substitué par un ou des groupes nitro ou carboxylique. X peut encore représenter un groupe alcoxycarbonyle, tel que le groupe méthoxycarbonyle.

Le radical R désigne la structure d'espacement (indiqué comme E dans la formule générale II ci-dessus) capable de porter simultanément les substituants Y' et S-S-H. Il devra être choisi de façon à ne pas comporter de fonctions susceptibles d'interférer au cours des réactions ultérieures avec les réactifs utilisés et les produits synthétisés. En particulier, le groupe R peut être un-groupe $-(CH_2)_n-$ avec n compris entre 1 et 10, ou encore un groupe :

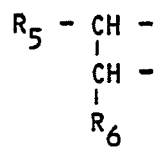

dans lequel
$R_6$ désigne l'hydrogène ou un groupe alkyle ayant de 1 à 8 atomes de carbone et $R_5$ désigne un substituant inerte vis-à-vis des réactifs utilisés ultérieurement, tel qu'un groupe carbamate

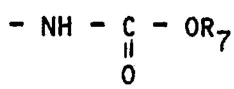

où $R_7$ désigne un groupe alkyle droit ou ramifié ayant de 1 à 5 atomes de carbone et notamment le groupe tertiobutyle.

La réaction du composé L-Y'-R-S-S-X avec la protéine P2 est effectuée en phase liquide homogène le plus souvent dans l'eau ou une solution tampon. Lorsque la solubilité des réactifs l'exige, il est possible d'ajouter au milieu réactionnel un solvant organique miscible à l'eau à une concentration finale pouvant atteindre 20 % en volume lorsqu'il s'agit d'un alcool tertiaire, tel que le butanol tertiaire ou 10 % en volume lorsqu'il s'agit du diméthylformamide ou du tétrahydrofuranne.

La réaction est effectuée à température ambiante pendant un temps variant de quelques minutes à quelques heures. Après quoi, une dialyse ou une filtration sur gel permet d'éliminer les produits de faible masse moléculaire et, en particulier, les excès de réactifs. Ce procédé permet d'introduire un nombre de groupements substituants par mole de protéine habituellement compris entre 1 et 15.

En utilisant de tels composés, le couplage avec la protéine P1 est réalisé par mise en présence des deux protéines en solution aqueuse de pH compris entre 6 et 8, à une température ne dépassant pas 30° C, pendant un temps variant de 1 heure à 24 heures. La solution aqueuse obtenue est éventuellement dialysée pour éliminer les produits de faible masse moléculaire, puis le conjugué peut être purifié par diverses méthodes connues.

B) Cas de la liaison thioéther

La préparation du conjugué consiste alors à faire réagir $P1'-(Z-Y-E)_n-SH$ avec la protéine P2 sur laquelle aura préalablement été introduit un ou plusieurs radicaux maléimide.

La réaction est alors représentée par le schéma suivant cité comme exemple :

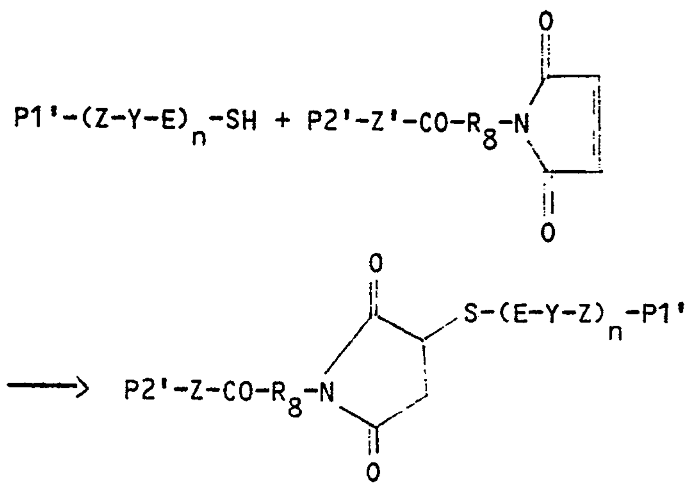

dans lequel :

- $R_8$ représente une structure d'espacement, aliphatique ou aromatique comportant de 1 à 15 atomes de carbone, inerte vis-à-vis des réactifs utilisés ultérieurement ;
- Z' représente des groupes variables selon le type de groupement fonctionnel de la protéine P2 intervenant dans le couplage.

  Ainsi, Z' = oxygène dans le cas d'un ester sur le phénol d'un résidu tyrosyle ;

  Z' = NH dans le cas du couplage d'un groupement carboxylique activé sur un groupement aminé de la protéine ;

  Z' = $NH-CH_2$ dans le cas de la réaction d'une chlorométhylcétone sur un groupement aminé de la protéine.

La protéine P2 substituée par le ou les groupes maléimide est obtenue à partir de la protéine P2 elle-même ou de la protéine P2 correctement protégée, par substitution de fonctions convenables de la protéine à l'aide d'un réactif lui-même porteur du groupement maléimide.

Parmi ces fonctions convenables, on retiendra particulièrement :

a) les fonctions aminées terminales des chaînes peptidiques ou les fonctions aminées latérales des résidus lysyle contenus dans la protéine. Dans ce cas, le réactif porteur du radical maléimide pourra être :

- soit un réactif de formule générale :

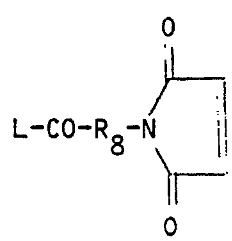

dans laquelle L-CO- représente :

. soit un groupe carboxylique, la réaction étant alors effectuée après activation de la fonction carboxylique en présence d'un agent de couplage, tel qu'un carbodiimide et notamment un dérivé soluble dans l'eau, tel que l'éthyl-1 (diméthylamino-3 propyl)-3 carbodiimide,

. soit un ester dit "activé" tel qu'un ester d'ortho- ou paranitro ou dinitrophényle, ou encore un ester de N-hydroxysuccinimide qui réagit directement avec les fonctions aminées pour les acyler.

La préparation de tels réactifs est notamment décrite dans Helvetica Chimica Acta 58, 531-541 (1975). D'autres réactifs de la même classe sont commercialement disponibles.

- soit un réactif de formule générale :

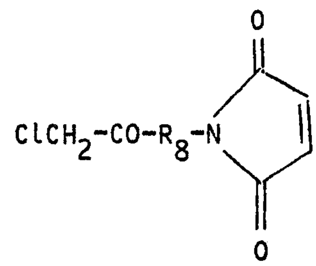

capable de réagir avec les groupements aminés de la protéine P2 selon le schéma :

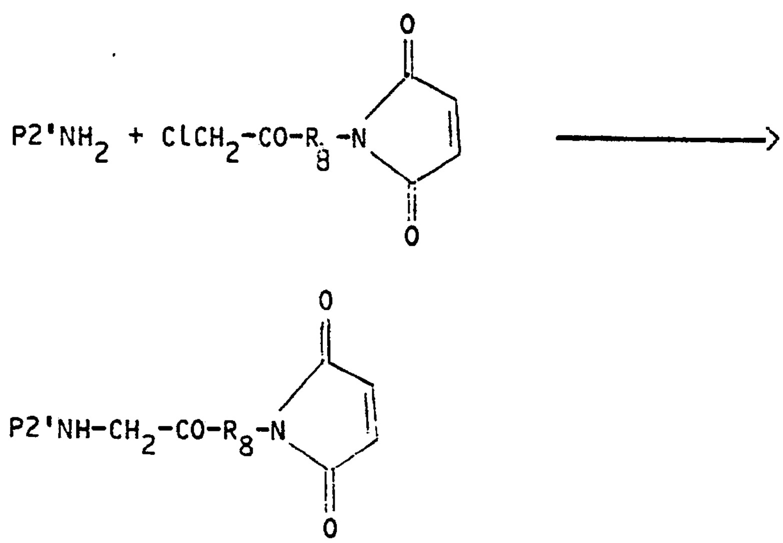

b) les fonctions phénol des radicaux tyrosyle contenus dans la protéine. Dans ce cas, le réactif porteur du radical maléimide pourra être un réactif de formule générale :

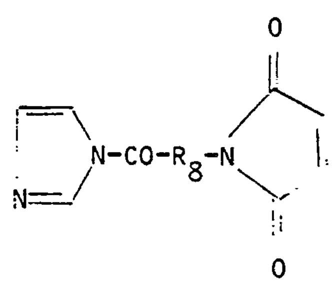

réagissant avec les groupes phénol de la protéine selon la réaction :

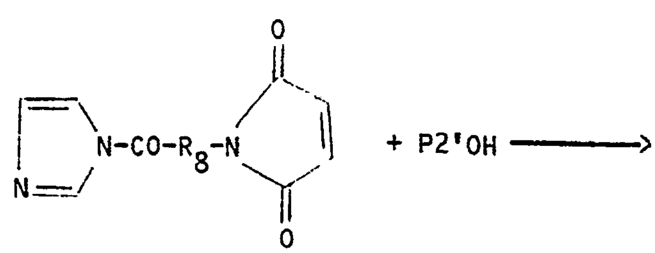

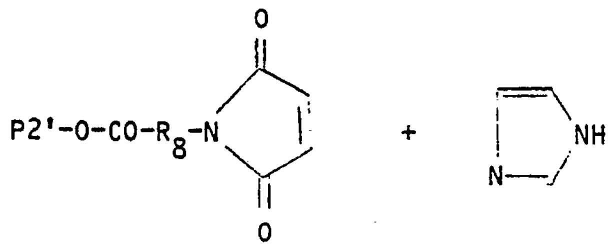

La réaction des réactifs porteurs de maléimide avec la protéine P2 est effectuée en phase liquide homogène le plus souvent dans l'eau ou une solution tampon. Lorsque la solubilité des réactifs l'exige, il est possible d'ajouter au milieu réactionnel un solvant organique miscible à l'eau à une concentration finale pouvant atteindre 20 % en volume lorsqu'il s'agit d'un alcool tertiaire tel que le butanol tertiaire ou 10 % en volume lorsqu'il s'agit du diméthylformamide ou du tétrahydrofuranne.

La réaction est effectuée à température ambiante pendant un temps variant de quelques minutes à quelques heures. Après quoi, une dialyse ou une filtration sur gel permet d'éliminer les produits de faible masse moléculaire et, en particulier, les excès de réactifs. Ce procédé permet d'introduire un nombre de groupements substituants par mole de protéine habituellement compris entre 1 et 15.

En utilisant de tels composés, le couplage avec la protéine P1 est réalisé par mise en présence des deux dérivés protéiques en solution aqueuse de pH compris entre 6 et 8, à une température ne dépassant pas 30 °C, pendant un temps variant de 1 heure à 24 heures. La solution obtenue est éventuellement dialysée pour éliminer les produits de faible masse moléculaire, puis le conjugué peut être purifié par diverses méthodes connues.

Parmi les composés de la présente invention, sont particulièrement convenables ceux de formule (II) dans laquelle W' représente un des groupements (a), (b), (c) et (d) ci-dessus, où E et E' représentent un groupe $-(CH_2)_p-$, p étant un nombre entier de 2 à 7 ou un groupe :

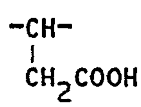

Ainsi, les dérivés de protéine particulièrement intéressants sont représentés par la formule statistique :

P2" - O - CO - E - G (VI)

dans laquelle :

- P2" représente le radical d'une protéine choisie parmi :
  . tout anticorps ou fragment d'anticorps ou toute immunoglobuline ou fragment d'immunoglobuline ou toute molécule dérivant des précédentes par modification artificielle de l'un quelconque de leurs groupements fonctionnels ;
  . la trichokirine ou toute molécule dérivant de ladite protéine par modification artificielle de l'un quelconque de ses groupements fonctionnels ;

  ledit radical étant la protéine privée d'un ou plusieurs groupes hydroxyles phénoliques des tyrosines ;
- l'atome d'oxygène est celui des groupes hydroxyles phénoliques manquant dans le radical P2" ;
- E et G sont tels que définis ci-dessus.

Particulièrement préférés sont les composés de formule (VI) dans laquelle E représente un groupe $-(CH_2)_p-$, où p est un nombre entier de 2 à 7 ou un groupe :

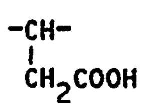

et G est un groupement de structure -S-S-X, où X est un radical activateur choisi parmi les groupes 2-pyridyle et 4-pyridyle non substitués ou substitués par un ou des halogènes ou des radicaux alkyle, carboxyle, alcoxycarbonyle, le groupe phényle non substitué ou substitué par un ou des halogènes ou des groupes nitro, alcoxy, carboxyle ou alcoxycarbonyle, ou un groupe alcoxycarbonyle.

Les produits de formule (VI) sont préparés en faisant réagir un produit de formule : P2"-OH, dans laquelle P2" est tel que défini ci-dessus et le groupe hydroxyle est l'hydroxyle phénolique manquant dans les tyrosines du radical P2", avec un composé de formule (VII) ci-dessous :

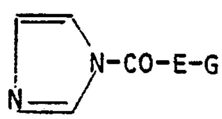

(VII)

à une température de 10 à 40 °C dans un solvant aqueux contenant éventuellement un solvant organique miscible à l'eau, tel que par exemple un solvant éthéré comme le dioxanne ou le tétrahydrofuranne.

Les exemples qui suivent permettent de mieux comprendre l'invention sans en limiter la portée.

L'anticorps monoclonal AT15E est dirigé contre l'antigène Thy 1.2 des lymphocytes murins. Cet anticorps est celui décrit dans Journal of Immunology 122, 2491-8, (1979) et a été obtenu à partir de l'hybridome décrit dans Hybridoma 1 (1), 13-17 (1981).

Dans les exemples, la trichokirine est désignée par l'abréviation TK.

**EXEMPLE 1 : ISOLEMENT DE LA TRICHOKIRINE**

A) Extrait brut

Des graines de Trichosanthes kirilowii (1,2 kg) sont broyées dans un appareil Ultraturrax avec 8 volumes de solution de chlorure de sodium 0,14 M contenant du tampon phosphate 5 mM, pH 7,5. L'extraction est poursuivie pendant une nuit à 4 °C sous agitation magnétique.

Après élimination des résidus grossiers, on procède à une centrifugation à 10 000 g à 0 °C pendant 45 minutes. Le surnageant est décanté à froid pour éliminer les graisses solidifiées puis les protéines sont précipitées par addition de sulfate d'ammonium jusqu'à saturation. Après centrifugation comme indiqué ci-dessus, le produit précipité est remis en suspension dans 1 litre de solution aqueuse 0,14 M de chlorure de sodium contenant du tampon phosphate 5 mM, pH 7,5. Le produit non dissous est éliminé par centrifugation dans les mêmes conditions que ci-dessus. Le surnageant est passé sur une colonne de Sephadex G 25 coarse (95 cm x 10 cm) équilibrée avec du tampon phosphate 5 mM, pH 7,0 à température ambiante et élué à la vitesse de 3 l/heure. Le premier pic est collecté.

B) Chromatographie d'échange d'ions

Les extraits combinés de 2 opérations (provenant à l'origine de 2,4 kg de graines) sont additionnés de chlorure de sodium jusqu'à une concentration finale de 30 mM et passés sur une colonne (17,5 cm x 5 cm) de CM-Sepharose Fast Flow équilibrée avec du tampon phosphate 30 mM, pH 7,0. Le produit non fixé est élué avec le même tampon. La protéine liée est éluée par un gradient contenant de 30 à 110 mM de chlorure de sodium dans le même tampon phosphate (volume 20 litres). Des fractions de 400 ml sont collectées à la vitesse de 1,2 litres par heure. L'absorbance à 280 nm et la conductivité de l'éluat sont contrôlées. Les fractions montrant une activité inhibitrice de la synthèse protéique sont réunies (3,6 l) et acidifiées à pH 5,8 par de l'acide acétique. On passe la solution sur une colonne de S-Sepharose Fast Flow (6,6 cm x 5 cm) équilibrée avec le tampon acétate de sodium 10 mM, pH 4,5. Après lavage avec le même tampon, la protéine fixée est éluée par du tampon phosphate 20 mM, pH 7,5 contenant du chlorure de sodium 0,5 M.

La fraction correspondant au pic contenant la protéine (200 ml) est recueillie et dialysée contre 3 fois 50 l d'eau distillée puis lyophilisée. On obtient ainsi 380 mg de trichokirine.

C) Filtration sur gel

La trichokirine obtenue ci-dessus est dissoute dans 50 ml de solution de chlorure de sodium 0,14 M. On élimine un insoluble par centrifugation à 20 000 g pendant 30 minutes. Le surnageant est passé sur une colonne de Sephadex G 50 coarse (95 cm x 5 cm) équilibrée avec le tampon phosphate 5 mM, pH 7,0 à

4° C On élue à la vitesse de 60 ml/heure. La solution correspondant au pic contenant la protéine est collectée, dialysée et lyophilisée comme indiqué ci-dessus.

On obtient ainsi 267 mg de trichokirine purifiée.

**EXEMPLE 2 : S-ACETYLMERCAPTOSUCCINOYL-TRICHOKIRINE ET MERCAPTOSUCCINOYL-TRICHOKIRINE**

A 5,8 mg de trichoyirine (0,215 micromole) dissoute dans 6 ml de tampon phosphate 125 mM, pH 7 (soit 0,973 mg/ml, 0,036 micromole environ/ml) sont additionnés 100 microlitres d'une solution d'anhydride S-acétylmercaptosuccinique (SAMSA) à 10,5 mg/ml (60,33 micromoles/ml) dans le diméthylformamide. L'incubation dure une heure puis le milieu réactionnel est purifié de l'excès de réactif par dialyse contre du tampon phosphate 125 mM, pH 7. On obtient ainsi 5,7 ml d'une solution de S-acétylmercaptosuccinoyl-trichokirine à 0,948 mg/ml. Par action de l'hydroxylamine et selon la méthode d'ELLMAN (Methods in Enzymology 25, 457, (1972)), on obtient la mercaptosuccinoyl-trichokirine contenant 0,7 groupes SH libres par mole de trichokirine (dosage spectrophotométrique). Examinée par électrophorèse en gradient de polyacrylamide, cette protéine modifiée présente une seule bande de poids moléculaire voisin de 28 000 ± 3 000 identique à celle de la protéine native.

**EXEMPLE 3 : CONJUGUE OBTENU PAR REACTION ENTRE L'ANTICORPS AT15E (ANTI-THY 1.2) ET LA MERCAPTOSUCCINOYL-TRICHOKIRINE**

A) Préparation de l'anticorps AT15E modifié

L'anticorps AT15E est un anticorps monoclonal dirigé contre l'antigène Thy 1.2 des lymphocytes murins. Cet anticorps est celui qui est décrit dans Journal of Immunology 122, 2491-8 (1979) et a été obtenu à partir de l'hybridome décrit dans Hybridoma 1(1); 13-17 (1981).

A 3,7 ml d'une solution d'anticorps AT15E à 3,55 mg/ml (soit 0,087 micromole) dans un tampon borate 0,1 M, pH 8,8 sont ajoutés 25 microlitres d'une solution de N-succinimidyl-3(pyridyl-2-disulfanyl)-3 propionate à 5,5 mg/ml dans l'éthanol.

L'incubation dure 30 minutes puis l'excès de réactif est éliminé par dialyse contre du tampon phosphate 125 mM, pH 7. Après dialyse, la solution proteique est centrifugée et l'on obtient 3,3 ml d'une solution à 3,4 mg/ml. Par dosage spectrophotométrique à 343 nm de la pyridine thione-2 libérée par échange avec le mercapto-2 éthanol, on constate que l'on a obtenu un anticorps portant 3,2 groupements disulfure mixte activé par mole d'anticorps.

B) Préparation de l'immunotoxine (conjugué)

A 1,9 ml de solution d'anticorps activé précédemment obtenue (soit 0,043 micromole), on ajoute 5 ml de la solution de trichokirine modifiée précédemment obtenue (soit 0,183 micromole) et on incube 20 heures à 25° C en présence de 350 microlitres d'une solution molaire de chlorhydrate d'hydroxylamine. La solution est centrifugée puis purifiée par filtration sur colonne de Sephadex G 200 avec mesure de la densité optique de l'effluent à 280 nm. Le regroupement des fractions contenant à la fois l'anticorps et la TK conduit à 20 ml d'une solution d'immunotoxine à 0,215 mg/ml (soit 4,3 mg). Cette solution contient 0,045 mg/ml de trichokirine modifiée couplée à l'anticorps.

Le taux de couplage moyen dans cette préparation est de 1,5 TK par mode d'anticorps. Le conjugué ainsi obtenu a la formule (IIIa) ci-dessus, dans laquelle P' est le radical de l'anticorps AT15E privé de fonctions amine, et m est 1,5.

TEST D'ACTIVITE DES IMMUNOTOXINES

L'une des propriétés de la trichokirine est d'inhiber la synthèse des protéines dans les cellules eucaryotes. Les tests réalisés sont donc des tests d'inhibition de la synthèse protéique:

- soit sur un modèle acellulaire,
- soit sur un modèle cellulaire.

A) Le modèle acellulaire

Le protocole in vitro utilise des fractions subcellulaires de foie de rat convenablement complémentées

et capables d'incorporer la 14C-phénylalanine en présence d'un ARN messager artificiel, l'acide polyuridylique.

Le mode opératoire employé pour préparer les fractions subcellulaires et pour mesurer l'incorporation de 14C-phénylalanine est une adaptation de la méthode décrite dans Biochimica et Biophysica Acta 312, 608-615 (1973), mettant en oeuvre à la fois une fraction microsomale et une fraction de cytosol des hépatocytes de rat. L'échantillon contenant la trichokirine est introduit sous la forme d'une solution convenablement diluée dans un tampon tris-HCl 50 mM, pH 7,6 contenant du mercapto-2 éthanol à 0,2 % et de la sérum albumine bovine à 15 microgrammes/ml. A partir des données de comptage, on calcule par rapport à un milieu témoin sans inhibiteur le pourcentage d'inhibition de l'incorporation de 14C-phénylalanine pour chaque milieu réactionnel.

L'ensemble des valeurs obtenues permet de déterminer la concentration de trichokirine (ou $CI_{50}$) qui inhibe 50 % de l'incorporation de la 14C-phénylalanine dans les conditions de l'essai. Pour la trichokirine native, on a ainsi déterminé que la $CI_{50}$ est égale à $7.10^{-11}$ M, tandis que celle de la trichokirine modifiée est de 8 à $10.10^{-11}$ mole/litre. La modification n'entraîne donc pas de perte importante d'activité de la trichokirine.

B) Cytotoxicité spécifique de l'immunotoxine à trichokirine sur modèle cellulaire

L'activité biologique de la trichokirine est d'inhiber la synthèse protéique cellulaire de systèmes eucaryotes. La technique utilisée met en oeuvre un modèle cellulaire dans lequel on mesure l'effet des substances étudiées sur l'incorporation de 14C-leucine dans les cellules cancéreuses en culture. Les cellules utilisées appartiennent à la lignée cellulaire T2, issue d'une leucémie T de souris qui exprime l'antigène de surface Thy 1.2. Les cellules sont incubées en présence de la substance à étudier à différentes concentrations puis, en fin d'incubation, on procède à la mesure du taux d'incorporation de 14C-leucine par les cellules ainsi traitées.

Cette mesure est effectuée selon une technique adaptée de la procédure décrite dans Journal of Biological Chemistry 249(II), 3557-3562, (1974), utilisant le traceur 14C-leucine pour la détermination du taux de synthèse protéique. La détermination de la radioactivité incorporée est effectuée ici sur les cellules entières isolées par filtration.

A partir de ces déterminations, on peut tracer les courbes effets doses présentant en abscisse la concentration molaire en trichokirine des substances étudiées et en ordonnée l'incorporation de 14C-leucine exprimée en pourcentage de l'incorporation des cellules témoins en l'absence de toute substance affectant la synthèse protéique.

On peut ainsi déterminer pour chaque substance étudiée la concentration qui inhibe 50 % de l'incorporation de 14C-leucine ou "concentration inhibitrice 50" ($CI_{50}$). Le tableau 2 représente les valeurs de $CI_{50}$ obtenues dans la même expérience avec l'immunotoxine à trichokirine d'une part, la trichokirine non couplée d'autre part.

TABLEAU 2

| Produits | Concentration inhibitrice 50 |
|---|---|
| IT à trichokirine | $2 \times 10^{-10}$ M |
| Trichokirine | $2 \times 10^{-6}$ M |

On peut constater que l'IT à trichokirine a une très forte activité cytotoxique qui est 10 000 fois supérieure à celle de la trichokirine non couplée.

**EXEMPLE 4 : CONJUGUE OBTENU PAR REACTION ENTRE L'ANTICORPS AT15E (ANTI-THY 1.2) ET LE (PYRIDYL-2 DISULFANYL)-3 PROPIONATE DE TRICHOKIRINE**

A) Préparation de l'anticorps modifié

A 20,1 mg d'une solution d'anticorps AT15E à 4,02 mg/ml (soit 0,134 micromoles) sont ajoutés 50 microlitres d'une solution d'anhydride S-acétylmercaptosuccinique (SAMSA) à 35 mg/ml dans le diméthylformamide. Le milieu réactionnel est incubé 2 heures 30 à 10 °C puis purifié de l'excès de réactif par dialyse contre du tampon phosphate 125 mM, pH 7.

On obtient 4,5 ml d'une solution à 3,7 mg/ml.

A 4,5 ml de cette solution d'anticorps sont ajoutés 200 microlitres d'une solution de chlorhydrate d'hydroxylamine 1 M. Après une incubation de 2 heures à 25 °C, le milieu réactionnel est purifié par dialyse de l'hydroxylamine contre du tampon phosphate 125 mM, pH 7.

Par dosage spectrophotométrique des groupements SH libérés par la méthode d'Ellmann, on constate que l'on a obtenu un IgG portant 3,8 groupements SH par mole d'anticorps.

B) Préparation du réactif de couplage

Il s'agit de l'imidazolide dérivé de l'acide (pyridyl-2 disulfanyl)-3 propionique dont la méthode de préparation a déjà été décrite dans la demande de brevet européenne n° 169 111.

215 mg d'acide (pyridyl-2 disulfanyl)-3 propionique sont dissous dans 0,5 ml de tétrahydrofuranne. A cette solution sont ajoutés 203 mg de CDI (carbonyl diimidazole). Le mélange est agité 15 min à 25 °C. On observe un dégagement gazeux de CO2. Le milieu réactionnel est utilisé directement et immédiatement sans purification.

C) Modification de la trichokirine

A 5 mg de TK dans 5 ml de tampon phosphate 125 mM, pH 7 (soit 0,185 micromoles) sont ajoutés 18,5 microlitres de la solution du réactif de couplage dans le THF. On laisse le mélange incuber 15 minutes à 25 °C puis le milieu réactionnel est purifié de l'excès de réactif par dialyse contre du tampon phosphate 125 mM, pH 7. Après centrifugation (15 min 15000 RPM), on obtient 4,4 ml d'une solution de TK modifiée à 0,94 mg/ml.

Par dosage spectrophotométrique à 343 nm de la pyridine thione-2 libérée par échange avec le mercapto-2 éthanol, on constate que l'on a obtenu une TK portant 0,6 groupement activateur par mole de TK.

D) Préparation de l'immunotoxine

A 0,5 ml de la solution d'anticorps précédemment obtenue (soit 0,012 micromoles), on ajoute 1,8 ml de la solution de TK (soit 0,063 micromoles). On laisse le mélange incuber 20 heures à 30 °C.

Le milieu réactionnel est purifié par passage sur une colonne ACA44, commercialisée par IBF-Rhône-Poulenc, et l'élution est suivie par mesure de la densité optique de l'effluent à 280 nm. L'élution est réalisée à l'aide de tampon PBS (phosphate salin). On obtient 6,6 ml d'une solution d'immunotoxine 0,146 mg/ml (soit 0,966 mg). Cette solution contient 0,022 mg/ml de TK modifiée. Le taux de couplage moyen de cette préparation est de une TK par mole d'anticorps. Le conjugué ainsi obtenu a la formule (IIIb) ci-dessus dans laquelle P' est le radical de l'anticorps AT15E privé de fonction amine, et m est 1.

E) Test d'activité de l'immunotoxine obtenue

La mesure de l'activité cytotoxique de l'immunotoxine est réalisée selon la même procédure que celle décrite dans l'exemple 3.

Le tableau 3 représente les valeurs de $CI_{50}$ obtenues dans la même expérience avec l'immunotoxine à trichokirine conjuguée par les tyrosines d'une part, la trichokirine non couplée d'autre part.

TABLEAU 3

| Produits | Concentration inhibitrice 50 |
|---|---|
| IT à trichokirine | $3 \times 10^{-10}$ M |
| Trichokirine | $3 \times 10^{-6}$ M |

On peut constater que l'IT préparée par la conjugaison de l'anticorps anti-Thy 1.2 à la trichokirine par ses résidus tyrosine a une très forte activité cytotoxique qui est 10 000 fois supérieure à celle de la trichokirine non couplée.

**EXEMPLE 5 : CONJUGUE OBTENU PAR REACTION ENTRE L'ANTICORPS ANTI-THY 1.2 AT15E SUBSTITUE PAR UN GROUPEMENT DISULFURE ACTIVE ET LA TK SUR LAQUELLE ON A INTRODUIT UN THIOL**

A) Préparation de la TK modifiée

A 7,25 mg de TK dissoute dans 8,5 ml de tampon phosphate 125 mM, pH 7 (soit 0,853 mg/ml) sont additionnés 168 microlitres d'une solution aqueuse de diméthyl-3,3-dithiobispropionimidate (DTBP). L'incubation dure 1 heure à 28° C puis le milieu réactionnel est purifié de l'excès de réactif par dialyse contre du tampon phosphate 125 mM, pH 7. Le pont disulfure du DTBP fixé à la protéine est ensuite réduit par le mercapto-2 éthanol (à la concentration finale de 1 pour cent pendant 1 heure à 30° C).

On poursuit la dialyse contre du tampon phosphate 125 mM, pH 7 comme précédemment. Après centrifugation, on obtient 7 ml d'une solution de TK modifiée à 0,794 mg/ml. Sur ce produit, la méthode d'Ellman permet de doser 0,6 groupement SH libre par mole de protéine.

B) Préparation de l'anticorps modifié

A 3 ml d'une solution d'anticorps AT15E à 4 mg/ml dans du tampon borate 0,1 M, pH 8,8 sont ajoutés 21 microlitres d'une solution contenant 5,2 mg de N-succinimidyl 3(pyridyl-2-disulfanyl)-3 propionate dissous dans 1 ml d'éthanol. L'incubation dure 1/2 heure à 28° C puis l'excès du réactif est éliminé par dialyse contre du tampon phosphate 125 mM, pH 7. Après dialyse, la solution protéique est centrifugée et l'on obtient 2,3 ml d'une solution à 3 mg/ml.

Par dosage spectrophotométrique à 343 nm de la pyridine thione-2 libérée par échange avec le mercapto-2 éthanol, on constate que l'on a obtenu un anti corps portant 3,8 groupements disulfure mixtes activés par mole d'anticorps.

C) Préparation de l'immunotoxine

A 2 ml de la solution d'anticorps modifié précédemment obtenue, on ajoute 5,4 ml de la solution de TK modifiée obtenue en A) et l'on incube 6 heures à 28° C. La solution est centrifugée puis purifiée par filtration sur une colonne de Sephacryl S200 HR avec mesure de la densité optique de l'effluent à 280 nm.

Le regroupement des fractions contenant à la fois l'anticorps et la TK conduit à 14 ml d'une solution d'immunotoxine à 0,09 mg/ml. Le taux de couplage moyen de cette préparation est de 1,5 mole de TK par mole d'anticorps.

Le conjugué ainsi obtenu a la formule II ci-dessus dans laquelle P' est le radical de l'anticorps AT15E privé de fonctions amine, W' est un groupement $-Z'-Y'-E'-S-S-(E-Y-Z)_n-$ où Z' est NH, Y' est CO, E' est $CH_2CH_2$, E est $CH_2CH_2$, Y est C=NH, Z est NH, n est 1, A' est le radical de la trichokirine telle que privée de fonctions amine et m est 1,5.

D) Test d'activité de l'immunotoxine obtenue

La mesure de l'activité de l'immunotoxine est réalisée selon la même procédure que celle décrite dans l'exemple 3. Le tableau 4 représente les valeurs de $CI_{50}$ obtenues dans la même expérience avec l'immunotoxine à trichokirine préparée comme ci-dessus d'une part et la trichokirine non couplée d'autre part.

TABLEAU 4

| Produits | Concentration inhibitrice 50 |
|---|---|
| IT à trichokirine | 8 à 10 x $10^{-11}$ M |
| Trichokirine | 3 x $10^{-6}$ M |

On peut constater que l'IT a une très forte activité cytotoxique qui est 30 000 fois supérieure à celle de la trichokirine non couplée.

Cette nouvelle classe d'immunotoxines peut être utilisée pour le traitement des affections cancéreuses ou non pour lesquelles les cellules cibles à détruire seraient reconnues par l'anticorps ou le fragment d'anticorps utilisé pour la préparation de l'immunotoxine. Les modalités optimales d'administration ainsi que la durée du traitement devront être déterminées dans chaque cas en fonction du sujet et de la nature de l'affection à traiter.

Les produits physiologiquement actifs de l'invention peuvent être formulés pour l'administration par toute voie convenable. Le plus souvent, le principe actif sera administré par injection et il sera formulé dans un liquide stérile apyrogène, de préférence de l'eau qui peut contenir des sels physiologiquement acceptables tels que des tampons ou du chlorure de sodium de façon à obtenir une solution isotonique.

Pour un traitement antitumoral, la dose journalière de trichokirine en tant que conjugué est de préférence comprise entre 1 et 100 mg, avantageusement de 5 à 50 mg et par exemple de 10 mg environ.

Les unités de dosage contenant la trichokirine sous forme de conjugué, par exemple des ampoules pour injection, contiendront donc habituellement de 1 à 100 mg de conjugué de trichokirine.

**Revendications**

**Revendications pour les Etats contractants suivants : AT, BE, CH, DE, FR, GB, IT, LI, LU, NL, SE**

**1.** Immunotoxine formée par couplage d'un anticorps avec la trichokirine, ayant la formule statistique suivante :

P' - W - A'

dans laquelle

P' représente le radical d'une protéine P qui est un anticorps ou un fragment d'anticorps tel quel ou chimiquement convenablement modifié, privée d'une ou plusieurs fonctions propres et dans lequel d'autres groupes fonctionnels sont éventuellement bloqués ;

A' représente le radical de la protéine A qui est la trichokirine telle quelle ou chimiquement convenablement modifiée, privée d'une ou plusieurs fonctions propres et dans lequel d'autres groupes fonctionnels sont éventuellement bloqués et

W représente une structure covalente bivalente contenant au moins un groupe thioéther ou un groupe disulfure dont un des atomes de soufre est soit choisi parmi ceux des cystéines de P ou A, soit lié aux fonctions propres de P et/ou A par des structures d'espacement portant un groupe fonctionnel lié auxdites fonctions propres de P et de A, la trichokirine étant une glycoprotéine

- ayant un poids moléculaire de 28 000 ± 3 000, déterminé par électrophorèse sur gel de polyacrylamide en présence de SDS (sodium dodécyl sulfate),
- de point isoélectrique ≧ 9
- de contenu en sucres neutres : 1,1 à 1,5 % en poids dont 0,3 à 1,2 % de mannose,
- de composition en acides aminés : nombre de résidus par mole de protéine ± 20 % :

| | | | | | | | | | | |
|---|---|---|---|---|---|---|---|---|---|---|
| Lys : 17,3 | ; | His : 1,1 | ; | Arg | : 6,7 | ; | Asx : 22,8 | ; |
| Thr : 18,9 | ; | Ser : 23,5 | ; | Glx | : 21,6 | ; | Pro : 8,0 | ; |
| Gly : 16,0 | ; | Ala : 21,1 | ; | 1/2 Cys | : 1,9 | ; | | |
| Val : 12,5 | ; | Met : 3,05 | ; | Ile | : 15,8 | ; | Leu : 24,3 | ; |
| Tyr : 12,1 | ; | Phe : 10,1 | ; | Trp | : n.d.. | | | |

tel que Asx est l'ensemble des résidus d'acide aspartique et d'asparagine

Glx est l'ensemble des résidus d'acide glutamique et de glutamine

1/2 Cys est le résidu de cystéine de la protéine native sous la forme d'acide cystéique déterminé lors de l'analyse,

et n.d. est non déterminé.

- de séquence amino-terminale :

1 10
Asp-Val-Ser-Phe-Ser-Leu-Ser-Gly-Gly-Gly-
16
Thr-Ala-Ser-Tyr-Glu-Lys

**2.** Immunotoxine formée par couplage d'un anticorps avec la trichokirine ayant la formule statistique suivante :

P' - W' - A' (II)

dans laquelle

P' et A' sont tels que définis dans la revendication 1 et W' représente une structure covalente choisie parmi :

(a) un groupement de formule :

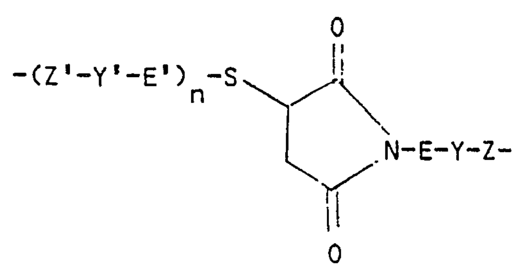

(b) un groupement de formule :

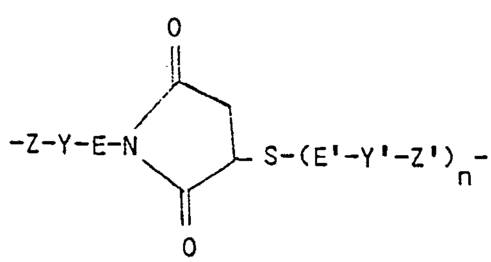

(c) un groupement de formule :

-Z'-Y'-E'-S-S-$(E\text{-}Y\text{-}Z)_n$-

(d) un groupement de formule :

-S-S-$(E\text{-}Y\text{-}Z)_n$-

où :

- Z et Z' identiques ou différents représentent les fonctions propres des protéines A et P, choisies parmi l'atome d'oxygène provenant de l'hydroxyle de l'un des résidus de tyrosine ; le groupe carbonyle provenant de l'un des carboxyles terminaux ou des carboxyles libres des acides aspartiques et/ou glutamiques de A et de P ; le groupe provenant de la structure dialdéhydique obtenue après oxydation de la structure glucidique de P ou le cas échéant de A par l'acide périodique ; et le groupe -NH- provenant de l'une des amines terminales de A et de P ou de l'une des amines en position epsilon de l'un des résidus de lysine ;
- Y et Y' représentent des groupes engagés dans une liaison covalente avec l'une quelconque des fonctions Z et Z' des protéines A et P ;
- E et E' représentent des structures d'espacement inertes ;
- n représente zéro ou 1.

**3.** Immunotoxine ayant la formule statistique suivante :

$P'(W' - A')_m$

dans laquelle
W' est tel que défini dans la revendication 2 ; m varie de 0,3 à 12 ; P' représente le radical d'une protéine P qui est un anticorps ou un fragment d'anticorps tel quel ou chimiquement convenablement modifié, privé d'une ou plusieurs fonctions propres et dans lequel les autres groupes fonctionnels sont éventuellement bloqués ; A' représente le radical de la protéine A qui est la trichokirine telle que définie dans la revendication 1.

**4.** Immunotoxine selon l'une quelconque des revendications 1 à 3, caractérisée en ce que la protéine A est la trichokirine modifiée représentée par la formule :

$$TK^{o} - (CO - \underset{\displaystyle CH_2COOH}{\underset{|}{CH}} - S - Q)_p$$

dans laquelle
$TK^o$ représente le radical de la trichokirine, Q représente l'hydrogène ou le groupe acétyle et p varie entre 0,2 et 3.

**5.** Immunotoxine selon la revendication 3, ayant la formule statistique suivante :

$$P'\text{-}(NH\text{-}CO\text{-}CH_2CH_2\text{-}S\text{-}S\text{-}\underset{\displaystyle CH_2COOH}{\underset{|}{CH}}\text{-}CO\text{-}NH\text{-}TK')_m$$

dans laquelle
P' et m sont tels que définis dans la revendication 3 et TK' est le radical de la trichokirine telle quelle, privée de fonctions amino.

**6.** Immunotoxine selon la revendication 3, ayant la formule statistique suivante :

$P'\text{-}(NH\text{-}CO\text{-}CH_2CH_2\text{-}S\text{-}S\text{-}CH_2CH_2\text{-}CO\text{-}O\text{-}TK'')_m$

dans laquelle
P' et m sont tels que définis dans la revendication 3 et TK" est le radical de la trichokirine telle quelle, privée de fonctions hydroxyle phénoliques.

**7.** Procédé pour la préparation d'une immunotoxine ayant la formule statistique suivante :

P' - W - A'

dans laquelle
P' représente le radical d'une protéine qui est un anticorps ou un fragment d'anticorps tel quel ou chimiquement convenablement modifié, privée d'une ou plusieurs fonctions propres et dans lequel les autres groupes fonctionnels sont éventuellement bloqués ; A' représente le radical de la protéine A qui est la trichokirine telle quelle ou chimiquement convenablement modifiée, privée d'une ou plusieurs fonctions propres et dans lequel les autres groupes fonctionnels sont éventuellement bloqués et W représente une structure covalente bivalente contenant au moins un groupe thioéther ou un groupe disulfure dont l'un des atomes de soufre est soit choisi parmi ceux des cystéines de P ou A, soit lié aux fonctions propres de P et/ou A par des structures d'espacement portant un groupe fonctionnel lié auxdites fonctions propres de P et/ou de A ; caractérisé en ce qu'on fait réagir en solution aqueuse et à la température ambiante une protéine P1, qui est indifféremment soit la trichokirine éventuellement modifiée, soit un anticorps ou fragment d'anticorps, porteuse d'au moins un groupe thiol libre attaché à ladite protéine P1 directement ou par l'intermédiaire d'une structure d'espacement, avec une protéine P2, différente de P1, qui est indifféremment soit la trichokirine éventuellement modifiée, soit un anticorps ou fragment d'anticorps, porteuse d'un groupement capable de couplage avec le thiol libre de la protéine P1, de façon à former une liaison thioéther ou disulfure.

**8.** Procédé selon la revendication 8, caractérisé en ce que l'on utilise comme protéine, P1 ou P2, un dérivé de protéine représenté par la formule statistique :

P2" - O - CO - E - G (VI)

dans laquelle :
- P2" représente le radical d'une protéine choisie parmi :
  . tout anticorps ou fragment d'anticorps ou toute immunoglobuline ou fragment d'immunoglobuline ou toute molécule dérivant des précédentes par modification artificielle de l'un quelconque de leurs groupements fonctionnels ;
  . la trichokirine ou toute molécule dérivant de cette protéine par modification artificielle de l'un quelconque de ses groupements fonctionnels ;

  ledit radical étant la protéine privée d'un ou plusieurs groupes hydroxyles phénoliques des tyrosines ;
- l'atome d'oxygène est celui des groupes hydroxyles phénoliques manquant dans le radical P2" ;
- E est tel que défini dans la revendication 2 et
- G représente un groupe :

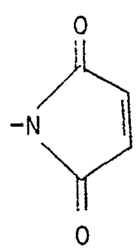

ou un groupe -S-S-X où X est un groupe activateur.

**9.** Procédé selon la revendication 7, caractérisé en ce que la protéine A est la trichokirine modifiée répondant à la formule :

$$TK^{O} - (CO - \underset{\displaystyle CH_2COOH}{\underset{|}{CH}} - S - Q)_p$$

dans laquelle
TK° représente le radical de la trichokirine, Q représente l'hydrogène ou le groupe acétyle et p varie entre 0,2 et 3.

**10.** Procédé selon la revendication 7, pour l'obtention d'une immunotoxine ayant la formule statistique :

$$P'(NH-CO-CH_2CH_2-S-S-\underset{\displaystyle CH_2COOH}{\underset{|}{CH}}-CO-NH-TK')_m \qquad (IIIa)$$

dans laquelle
P' et m sont tels que définis dans la revendication 3 et TK' est le radical de la trichokirine telle quelle, privée de fonctions amino, caractérisé en ce qu'il consiste à faire réagir, en solution aqueuse et à température ambiante, une protéine P1 qui est un anticorps ou un fragment d'anticorps portant au moins un groupe thiol libre lié à ladite protéine P1 par l'intermédiaire d'une structure d'espacement de formule $-NH-CO-CH_2-CH_2-$, avec la mercaptosuccinoyl-trichokirine comme protéine P2.

**11.** Procédé selon la revendication 7, pour l'obtention d'une immunotoxine ayant la formule statistique :

$P'-(NH-CO-CH_2CH_2-S-S-CH_2CH_2-CO-O-TK")_m$ (IIIb)

dans laquelle
P' et m sont tels que définis dans la revendication 3, et TK" est le radical de la trichokirine telle quelle privée de fonctions hydroxyle phénoliques, caractérisé en ce qu'il consiste à faire réagir, en solution aqueuse et à température ambiante, une protéine P1 qui est un anticorps ou un fragment d'anticorps portant au moins un groupe thiol libre lié à ladite protéine P1 par l'intermédiaire d'une structure d'espacement de formule $-NH-CO-CH_2-CH_2$ avec le (pyridyl-2 disulfanyl)-3 propionate de trichokirine comme protéine P2.

**12.** Dérivés de protéine représentés par la formule statistique :

P2" - O - CO - E - G (VI)

dans laquelle :
- P2" représente le radical
  . de la trichokirine ou toute molécule dérivant de ladite protéine par modification artificielle de l'un quelconque de ses groupements fonctionnels ;

  ledit radical étant la protéine privée d'un ou plusieurs groupes hydroxyles phénoliques des tyrosines ;
- l'atome d'oxygène est celui des groupes hydroxyles phénoliques manquant dans le radical P2" ;
- E est tel que défini dans la revendication 2, et
- G représente un groupe

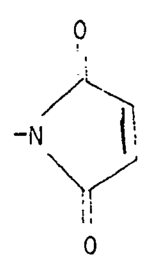

ou un groupe -S-S-X où X est un groupe activateur.

**13.** Procédé pour l'obtention des dérivés de protéine selon la revendication 12, caractérisé en ce qu'il consiste à faire réagir un produit de formule : P2"-OH, dans laquelle P2" est tel que défini ci-dessus et le groupe hydroxyle étant l'hydroxyle phénolique manquant dans les tyrosines du radical P2", avec un composé de formule (VII) ci-dessous :

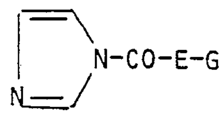

(VII)

à une température de 10 à 40 °C dans un solvant aqueux contenant éventuellement un solvant organique miscible à l'eau, tel que par exemple un solvant éthéré comme le dioxanne ou le tétrahydrofuranne.

**14.** Compositions pharmaceutiques contenant en tant que principe actif une immunotoxine selon l'une quelconque des revendications 1 à 6.

**Revendications pour l'Etat contractant suivant : ES**

**1.** Procédé pour l'obtention d'une immunotoxine ayant la formule statistique suivante :

P' - W - A' (I)

dans laquelle
P' représente le radical d'une protéine qui est un anticorps ou un fragment d'anticorps tel quel ou chimiquement convenablement modifié, privée d'une ou plusieurs fonctions propres et dans lequel les autres groupes fonctionnels sont éventuellement bloqués ; A' représente le radical de la protéine A qui est la trichokirine telle quelle ou chimiquement convenablement modifiée, privée d'une ou plusieurs fonctions propres et dans lequel les autres groupes fonctionnels sont éventuellement bloqués et W représente une structure covalente bivalente contenant au moins un groupe thioéther ou un groupe disulfure dont l'un des atomes de soufre est soit choisi parmi ceux des cystéines de P ou A, soit lié aux fonctions propres de P et/ou A par des structures d'espacement portant un groupe fonctionnel lié auxdites fonctions propres de P et/ou de A ; caractérisé en ce qu'on fait réagir en solution aqueuse et à la température ambiante une protéine P1, qui est indifféremment soit la trichokirine éventuellement modifiée, soit un anticorps ou fragment d'anticorps, porteuse d'au moins un groupe thiol libre attaché à ladite protéine P1 directement ou par l'intermédiaire d'une structure d'espacement, avec une protéine P2, différente de P1, qui est indifféremment soit la trichokirine éventuellement modifiée, soit un anticorps ou fragment d'anticorps, porteuse d'un groupement capable de couplage avec le thiol libre de la protéine P1, de façon à former une liaison thioéther ou disulfure, la trichokirine etant une glycoprotéine

- ayant un poids moléculaire de 28 000 ± 3 000, déterminé par électrophorèse sur gel de polyacrylamide en présence de SDS (sodium dodécyl sulfate),
- de point isoélectrique ≧ 9
- de contenu en sucres neutres : 1,1 à 1,5 % en poids dont 0,3 à 1,2 % de mannose,
- de composition en acides aminés : nombre de résidus par mole de protéine ± 20 % :

| | | | |
|---|---|---|---|
| Lys : 17,3 ; | His : 1,1 ; | Arg : 6,7 ; | Asx : 22,8 ; |
| Thr : 18,9 ; | Ser : 23,5 ; | Glx : 21,6 ; | Pro : 8,0 ; |
| Gly : 16,0 ; | Ala : 21,1 ; | 1/2 Cys : 1,9 ; | |
| Val : 12,5 ; | Met : 3,05 ; | Ile : 15,8 ; | Leu : 24,3 ; |
| Tyr : 12,1 ; | Phe : 10,1 ; | Trp : n.d.. | |

tel que Asx est l'ensemble des résidus d'acide aspartique et d'asparagine

Glx est l'ensemble des résidus d'acide glutamique et de glutamine

1/2 Cys est le résidu de cystéine de la protéine native sous la forme d'acide cystéique déterminé lors de l'analyse,

et n.d. est non déterminé.

- de séquence amino-terminale :

1 10
Asp-Val-Ser-Phe-Ser-Leu-Ser-Gly-Gly-Gly-
16
Thr-Ala-Ser-Tyr-Glu-Lys

**2.** Procédé pour l'obtention d'une immunotoxine ayant la formule statistique suivante :

P' - W' - A' (II)

dans laquelle
P' et A' sont tels que définis dans la revendication 1 et W' représente une structure covalente choisie parmi :

(a) un groupement de formule :

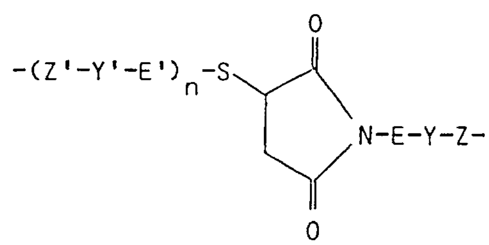

(b) un groupement de formule :

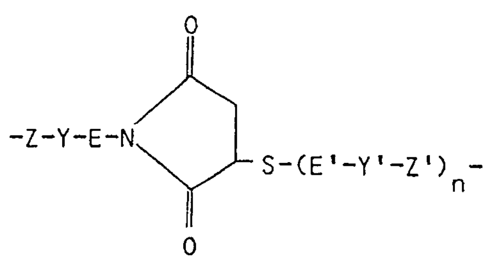

(c) un groupement de formule :

-Z'-Y'-E'-S-S-$(E-Y-Z)_n$-

(d) un groupement de formule :

-S-S-$(E-Y-Z)_n$-

où :

- Z et Z' identiques ou différents représentent les fonctions propres des protéines A et P, choisies parmi l'atome d'oxygène provenant de l'hydroxyle de l'un des résidus de tyrosine ; le groupe

carbonyle provenant de l'un des carboxyles terminaux ou des carboxyles libres des acides aspartiques et/ou glutamiques de A et de P ; le groupe provenant de la structure dialdéhydique obtenue après oxydation de la structure glucidique de P, ou le cas échéant de A par l'acide periodique ; et le groupe -NH- provenant de l'une des amines terminales de A et de P ou de l'une des amines en position epsilon de l'un des résidus de lysine ;

- Y et Y' représentent des groupes engagés dans une liaison covalente avec l'une quelconque des fonctions Z et Z' des protéines A et P ;
- E et E' représentent des structures d'espacement inertes ;
- n représente zéro ou 1

caractérisé en ce qu'on fait réagir en solution aqueuse et à la température ambiante une protéine P1, qui est indifféremment soit la trichokirine éventuellement modifiée, soit un anticorps ou fragment d'anticorps, porteuse d'au moins un groupe thiol libre attaché à ladite protéine P1 directement ou par l'intermédiaire d'une structure d'espacement, avec une protéine P2, différente de P1, qui est indifféremment soit la trichokirine éventuellement modifiée, soit un anticorps ou fragment d'anticorps, porteuse d'un groupement capable de couplage avec le thiol libre de la protéine P1, de façon à former une liaison thioéther ou disulfure.

**3.** Procédé pour l'obtention d'une immunotoxine ayant la formule statistique suivante :

$$P'(W' - A')_m$$

dans laquelle
W' est tel que défini dans la revendication 2 ; m varie entre 0,3 et 12 ; P' et A' sont tels que définis dans la revendication 1, caractérisé en ce qu'on fait réagir en solution aqueuse et à la température ambiante une protéine P1, qui est indifféremment soit la trichokirine éventuellement modifiée, soit un anticorps ou fragment d'anticorps, porteuse d'au moins un groupe thiol libre attaché à ladite protéine P1 directement ou par l'intermédiaire d'une structure d'espacement, avec une protéine P2, différente de P1, qui est indifféremment soit la trichokirine éventuellement modifiée, soit un anticorps ou fragment d'anticorps, porteuse d'un groupement capable de couplage avec le thiol libre de la protéine P1, de façon à former une liaison thioéther ou disulfure.

**4.** Procédé selon la revendication 3 pour l'obtention d'une immunotoxine ayant la formule statistique suivante :

$$P'-(NH-CO-CH_2CH_2-S-S-\underset{\displaystyle CH_2COOH}{\underset{|}{CH}}-CO-NH-TK')_m \qquad (IIIa)$$

dans laquelle
P' et m sont tels que définis dans la revendication 3 et TK' est le radical de la trichokirine telle quelle, privée de fonctions amino, caractérisé en ce qu'il consiste à faire réagir en solution aqueuse et à la température ambiante une protéine P1 qui est un anticorps ou un fragment d'anticorps porteur d'au moins un groupe thiol libre attaché à ladite protéine P1 directement ou par l'intermédiaire d'une structure d'espacement avec une protéine P2 qui est la mercaptosuccinoyl-trichokirine.

**5.** Procédé selon la revendication 3 pour l'obtention d'une immunotoxine ayant la formule statistique suivante :

$$P'-(NH-CO-CH_2CH_2-S-S-CH_2CH_2-CO-O-TK'')_m \qquad (IIIb)$$

dans laquelle
P' et m sont tels que définis dans la revendication 3 et TK" est le radical de la trichokirine telle quelle, privée de fonctions hydroxyle phénoliques, caractérisé en ce qu'il consiste à faire réagir en solution aqueuse et à la température ambiante une protéine P1 qui est un anticorps ou un fragment d'anticorps porteur d'au moins un groupe thiol libre attaché à ladite protéine P1 directement ou par l'intermédiaire d'une structure d'espacement avec une protéine P2 qui est le (pyridyl-2 disulfanyl)-3 propionate de trichokirine.

6. Procédé selon l'une quelconque des revendications 1 à 3, caractérisé en ce que la protéine A est la trichokirine modifiée de formule :

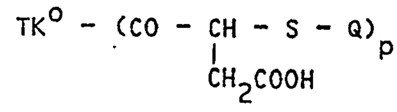

dans laquelle
TK° représente le radical de la trichokirine, Q représente l'hydrogène ou le groupe acétyle et p varie entre 0,2 et 3.

7. Procédé selon l'une quelconque des revendications 1 à 6, caractérisé en ce que l'on utilise comme protéine P1 ou P2, des dérivés de protéines de formule statistique :

P2" - O - CO - E - G (VI)

dans laquelle
- P2" représente le radical d'une protéine choisie parmi :
  . tout anticorps ou fragment d'anticorps ou toute immunoglobuline ou fragment d'immunoglobuline ou toute molécule dérivant des précédentes par modification artificielle de l'un quelconque de leurs groupements fonctionnels ;
  . la trichokirine ou toute molécule dérivant de cette protéine par modification artificielle de l'un quelconque de ses groupements fonctionnels ;

  ledit radical étant la protéine privée d'un ou plusieurs groupes hydroxyles phénoliques des tyrosines ;
- l'atome d'oxygène est celui des groupes hydroxyles phénoliques manquant dans le radical P2" ;
- E est tel que défini dans la revendication 2, et
- G représente un groupe :

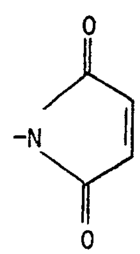

ou un groupe -S-S-X où X est un groupe activateur.

8. Procédé pour l'obtention de dérivés de protéine de formule statistique :

P2" - O - CO - E - G (VI)

dans laquelle
E et G sont tels que définis dans la revendication 7, P2" représente le radical de la trichokirine ou toute molécule dérivant de ladite protéine par modification artificielle de l'un quelconque de ses groupements fonctionnels, ledit radical etant la protéine privée d'une ou plusieurs groupes hydroxyles phénoliques des tyrosines ;
caractérisé en ce qu'il consiste à faire réagir un produit de formule : P2"-OH, dans laquelle P2" est tel que défini ci-dessus et le groupe hydroxyle étant l'hydroxyle phénolique manquant dans les tyrosines du radical P2", avec un composé de formule (VII) ci-dessous :

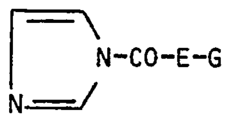

(VII)

dans laquelle
E et G sont tels que définis ci-dessus à une température de 10 à 40 °C dans un solvant aqueux contenant éventuellement un solvant organique miscible à l'eau, tel que par exemple un solvant éthéré comme le dioxanne ou le tétrahydrofuranne.

**Revendications pour l'Etat contractant suivant : GR**

**1.** Immunotoxine formée par couplage d'un anticorps avec la trichokirine, ayant la formule statistique suivante :

P' - W - A'

dans laquelle
P' représente le radical d'une protéine P qui est un anticorps ou un fragment d'anticorps tel quel ou chimiquement convenablement modifié, privée d'une ou plusieurs fonctions propres et dans lequel d'autres groupes fonctionnels sont éventuellement bloqués ;
A' représente le radical de la protéine A qui est la trichokirine telle quelle ou chimiquement convenablement modifiée, privée d'une ou plusieurs fonctions propres et dans lequel d'autres groupes fonctionnels sont éventuellement bloqués et
W représente une structure covalente bivalente contenant au moins un groupe thioéther ou un groupe disulfure dont un des atomes de soufre est soit choisi parmi ceux des cystéines de P ou A, soit lié aux fonctions propres de P et/ou A par des structures d'espacement portant un groupe fonctionnel lié auxdites fonctions propres de P et de A, la trichokirine étant une glycoprotéine

- ayant un poids moléculaire de 28 000 ± 3 000, déterminé par électrophorèse sur gel de polyacrylamide en présence de SDS (sodium dodécyl sulfate),
- de point isoélectrique ≧ 9
- de contenu en sucres neutres : 1,1 à 1,5 % en poids dont 0,3 à 1,2 % de mannose,
- de composition en acides aminés : nombre de résidus par mole de protéine ± 20 % :

| | | | |
|---|---|---|---|
| Lys : 17,3 ; | His : 1,1 ; | Arg : 6,7 ; | Asx : 22,8 ; |
| Thr : 18,9 ; | Ser : 23,5 ; | Glx : 21,6 ; | Pro : 8,0 ; |
| Gly : 16,0 ; | Ala : 21,1 ; | 1/2 Cys : 1,9 ; | |
| Val : 12,5 ; | Met : 3,05 ; | Ile : 15,8 ; | Leu : 24,3 ; |
| Tyr : 12,1 ; | Phe : 10,1 ; | Trp : n.d.. | |

tel que Asx est l'ensemble des résidus d'acide aspartique et d'asparagine
Glx est l'ensemble des résidus d'acide glutamique et de glutamine
1/2 Cys est le résidu de cystéine de la protéine native sous la forme d'acide cystéique déterminé lors de l'analyse,
et n.d. est non déterminé.

- de séquence amino-terminale :

1 10
Asp-Val-Ser-Phe-Ser-Leu-Ser-Gly-Gly-Gly-
16
Thr-Ala-Ser-Tyr-Glu-Lys

**2.** Immunotoxine formée par couplage d'un anticorps avec la trichokirine, ayant la formule statistique suivante :

P' - W' - A' (II)

dans laquelle
P' et A' sont tels que définis dans la revendication 1 et W' représente une structure covalente choisie parmi :

(a) un groupement de formule :

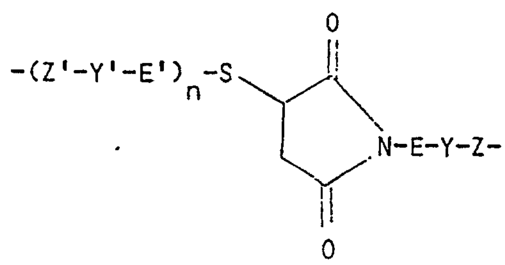

(b) un groupement de formule :

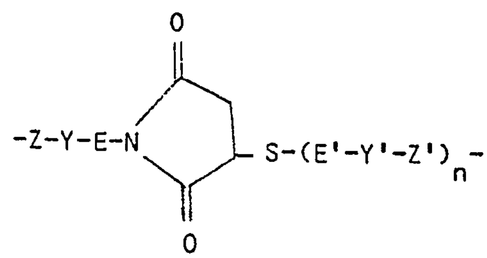

(c) un groupement de formule :

$-Z'-Y'-E'-S-S-(E-Y-Z)_n-$

(d) un groupement de formule :

$-S-S-(E-Y-Z)_n-$

où :

- Z et Z' identiques ou différents représentent les fonctions propres des protéines A et P, choisies parmi l'atome d'oxygène provenant de l'hydroxyle de l'un des résidus de tyrosine ; le groupe carbonyle provenant de l'un des carboxyles terminaux ou des carboxyles libres des acides aspartiques et/ou glutamiques de A et de P ; le groupe provenant de la structure dialdéhydique obtenue après oxydation de la structure glucidique de P ou le cas échéant de A par l'acide périodique ; et le groupe -NH- provenant de l'une des amines terminales de A et de P ou de l'une des amines en position epsilon de l'un des résidus de lysine ;
- Y et Y' représentent des groupes engagés dans une liaison covalente avec l'une quelconque des fonctions Z et Z' des protéines A et P ;
- E et E' représentent des structures d'espacement inertes ;
- n représente zéro ou 1.

**3.** Immunotoxine ayant la formule statistique suivante :

$P'(W' - A')_m$

dans laquelle

W' est tel que défini dans la revendication 2 ; m varie de 0,3 à 12 ; P' représente le radical d'une protéine P qui est un anticorps ou un fragment d'anticorps tel quel ou chimiquement convenablement modifié, privé d'une ou plusieurs fonctions propres et dans lequel les autres groupes fonctionnels sont éventuellement bloqués ; A' représente le radical de la protéine A qui est la trichokirine telle que définie dans la revendication 1.

4. Immunotoxine selon l'une quelconque des revendications 1 à 3, caractérisée en ce que la protéine A est la trichokirine modifiée représentée par la formule :

$$TK^{o}-(CO-\underset{\displaystyle CH_2COOH}{\underset{|}{CH}}-S-Q)_p$$

dans laquelle
$TK^o$ représente le radical de la trichokirine, Q représente l'hydrogène ou le groupe acétyle et p varie entre 0,2 et 3.

5. Immunotoxine selon la revendication 3, ayant la formule statistique suivante :

$$P'-(NH-CO-CH_2CH_2-S-S-\underset{\displaystyle CH_2COOH}{\underset{|}{CH}}-CO-NH-TK')_m$$

dans laquelle
P' et m sont tels que définis dans la revendication 4 et TK' est le radical de la trichokirine telle quelle, privée de fonctions amino.

6. Immunotoxine selon la revendication 3, ayant la formule statistique suivante :

$P'-(NH-CO-CH_2CH_2-S-S-CH_2CH_2-CO-O-TK'')_m$

dans laquelle
P' et m sont tels que définis dans la revendication 3 et TK" est le radical de la trichokirine telle quelle, privée de fonctions hydroxyle phénoliques.

7. Procédé pour la préparation d'une immunotoxine ayant la formule statistique suivante :

P' - W - A'

dans laquelle
P' représente le radical d'une protéine qui est un anticorps ou un fragment d'anticorps tel quel ou chimiquement convenablement modifié, privée d'une ou plusieurs fonctions propres et dans lequel les autres groupes fonctionnels sont éventuellement bloqués ; A' représente le radical de la protéine A qui est la trichokirine telle quelle ou chimiquement convenablement modifiée, privée d'une ou plusieurs fonctions propres et dans lequel les autres groupes fonctionnels sont éventuellement bloqués et W représente une structure covalente bivalente contenant au moins un groupe thioéther ou un groupe disulfure dont l'un des atomes de soufre est soit choisi parmi ceux des cystéines de P ou A, soit lié aux fonctions propres de P et/ou A par des structures d'espacement portant un groupe fonctionnel lié auxdites fonctions propres de P et/ou de A ; caractérisé en ce qu'on fait réagir en solution aqueuse et à la température ambiante une protéine P1, qui est indifféremment soit la trichokirine éventuellement modifiée, soit un anticorps ou fragment d'anticorps, porteuse d'au moins un groupe thiol libre attaché à ladite protéine P1 directement ou par l'intermédiaire d'une structure d'espacement, avec une protéine P2, différente de P1, qui est indifféremment soit la trichokirine éventuellement modifiée, soit un anticorps ou fragment d'anticorps, porteuse d'un groupement capable de couplage avec le thiol libre de

la protéine P1, de façon à former une liaison thioéther ou disulfure.

**8.** Procédé selon la revendication 8, caractérisé en ce que l'on utilise comme protéine, P1 ou P2, un dérivé de protéine représenté par la formule statistique :

P2" - O - CO - E - G (VI)

dans laquelle :

- P2" représente le radical d'une protéine choisie parmi :
  . tout anticorps ou fragment d'anticorps ou toute immunoglobuline ou fragment d'immunoglobuline ou toute molécule dérivant des précédentes par modification artificielle de l'un quelconque de leurs groupements fonctionnels ;
  . la trichokirine ou toute molécule dérivant de cette protéine par modification artificielle de l'un quelconque de ses groupements fonctionnels ;
  
  ledit radical étant la protéine privée d'un ou plusieurs groupes hydroxyles phénoliques des tyrosines ;
- l'atome d'oxygène est celui des groupes hydroxyles phénoliques manquant dans le radical P2" ;
- E est tel que défini dans la revendication 2 et
- G représente un groupe :

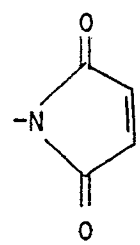

ou un groupe -S-S-X où X est un groupe activateur.

**9.** Procédé selon la revendication 7, caractérisé en ce que la protéine A est la trichokirine modifiée répondant à la formule :

$$TK^{o} - (CO - \underset{CH_2COOH}{CH} - S - Q)_p$$

dans laquelle
TK° représente le radical de la trichokirine, Q représente l'hydrogène ou le groupe acétyle et p varie entre 0,2 et 3.

**10.** Procédé selon la revendication 7, pour l'obtention d'une immunotoxine ayant la formule statistique :

$$P'(NH-CO-CH_2CH_2-S-S-\underset{CH_2COOH}{CH}-CO-NH-TK')_m \qquad (IIIa)$$

dans laquelle
P' et m sont tels que définis dans la revendication 3 et TK' est le radical de la trichokirine telle quelle, privée de fonctions amino, caractérisé en ce qu'il consiste à faire réagir, en solution aqueuse et à température ambiante, une protéine P1 qui est un anticorps ou un fragment d'anticorps portant au moins un groupe thiol libre lié à ladite protéine P1 par l'intermédiaire d'une structure d'espacement de formule $-NH-CO-CH_2-CH_2-$, avec la mercaptosuccinoyl-trichokirine comme protéine P2.

**11.** Procédé selon la revendication 7, pour l'obtention d'une immunotoxine ayant la formule statistique :

$$P'\text{-}(NH\text{-}CO\text{-}CH_2CH_2\text{-}S\text{-}S\text{-}CH_2CH_2\text{-}CO\text{-}O\text{-}TK")_m \quad (IIIb)$$

dans laquelle
P' et m sont tels que définis dans la revendication 3, et TK" est le radical de la trichokirine telle quelle privée de fonctions hydroxyle phénoliques, caractérisé en ce qu'il consiste à faire réagir, en solution aqueuse et à température ambiante, une protéine P1 qui est un anticorps ou un fragment d'anticorps portant au moins un groupe thiol libre lié à ladite protéine P1 par l'intermédiaire d'une structure d'espacement de formule $-NH\text{-}CO\text{-}CH_2\text{-}CH_2$ avec le (pyridyl-2 disulfanyl)-3 propionate de trichokirine comme protéine P2.

**12.** Dérivés de protéine représentés par la formule statistique :.

P2" - O - CO - E - G (VI)

dans laquelle :
- P2" représente le radical
  . de la trichokirine ou toute molécule dérivant de ladite protéine par modification artificielle de l'un quelconque de ses groupements fonctionnels ;

  ledit radical étant la protéine privée d'un ou plusieurs groupes hydroxyles phénoliques des tyrosines ;
- l'atome d'oxygène est celui des groupes hydroxyles phénoliques manquant dans le radical P2" ;
- E est tel que défini dans la revendication 2, et
- G représente un groupe

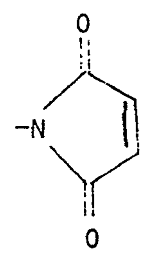

ou un groupe -S-S-X où X est un groupe activateur.

**13.** Procédé pour l'obtention des dérivés de protéine selon la revendication 12, caractérisé en ce qu'il consiste à faire réagir un produit de formule : P2"-OH, dans laquelle P2" est tel que défini ci-dessus et le groupe hydroxyle étant l'hydroxyle phénolique manquant dans les tyrosines du radical P2", avec un composé de formule (VII) ci-dessous :

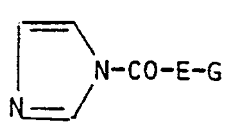

(VII)

à une température de 10 à 40 °C dans un solvant aqueux contenant éventuellement un solvant organique miscible à l'eau, tel que par exemple un solvant éthéré comme le dioxanne ou le tétrahydrofuranne.

**Claims**

**Claims for the following Contracting States : AT, BE, CH, DE, FR, GB, IT, LI, LU, NL, SE**

**1.** An immunotoxin formed by the coupling of an antibody with trichokirin, having the following statistical formula:

P'-W-A'

in which P' represents the radical of a protein P, which is an antibody or antibody fragment, as such or appropriately chemically modified, from which one or more of its own functions have been removed and in which other functional groups are optionally blocked, A' represents the radical of a protein A which is trichokirin, as such or appropriately chemically modified, from which one or more of its own functions have been removed and in which other functional groups are optionally blocked, and W represents a divalent covalent structure containing at least one thioether group or one disulfide group in which either one of the sulfur atoms is that of the cysteines of P or A, or it is bonded to the own functions of P and/or A by spacing structures carrying a functional group bonded to the own functions of P and/or A, the trichokirin being a glycoprotein:

- having a molecular weight of 28,000 ± 3,000 as determined by electrophoresis on polyacrylamide gel in the presence of SDS (sodium dodecyl-sulfate);
- with an isoelectric point ≧ 9;
- with a content of neutral sugars of 1.1 to 1.5% by weight, including 0.3 to 1.2% of mannose;
- having the following amino acid composition, expressed as the number of residues per mol of protein ± 20%:

| | | | |
|---|---|---|---|
| Lys : 17.3 ; | His : 1.1 ; | Arg : 6.7 ; | Asx : 22.8 ; |
| Thr : 18.9 ; | Ser : 23.5 ; | Glx : 21.6 ; | Pro : 8.0 ; |
| Gly : 16.0 ; | Ala : 21.1 ; | 1/2 Cys : 1.9 ; | |
| Val : 12.5 ; | Met : 3.05 ; | Ile : 15.8 ; | Leu : 24.3 ; |
| Tyr : 12.1 ; | Phe : 10.1 ; | Trp : n.d.. | |

where Asx is the aspartic acid and asparagine residues together, Glx is the glutamic acid and glutamine residues together, 1/2 Cys is determined as being the cysteic residues of the native protein in the form of the cysteic acid determined during the analysis and n.d. means "not determined"; and

- it has the following terminal amino sequence:

1 10
Asp-Val-Ser-Phe-Ser-Leu-Ser-Gly-Gly-Gly-
16
Thr-Ala-Ser-Tyr-Glu-Lys

2. An immunotoxin formed by coupling an antibody with the trichokirin having the following statistical formula:

P'-W'-A' II

in which:
P' and A' are as defined in claim 1 and W' represents a covalent structure chosen from:
a) a group of formula:

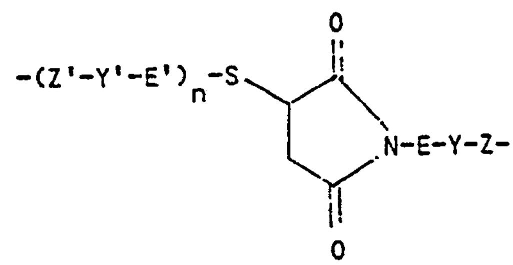

b) a group of formula:

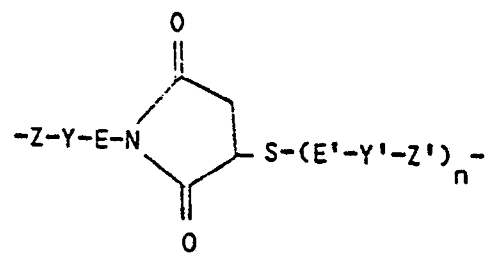

c) a group of formula:

$-Z'-Y'-E'-S-S-(E-Y-Z)_n-$

d) a group of formula:

$-S-S-(E-Y-Z)_n-$

in which:

- Z and Z', which are identical or different, represent the own functions of the proteins A and P, chosen from the oxygen atom originating from the hydroxyl of one of the tyrosine residues; the carbonyl group originating from one of the terminal carboxyls or the free carboxyls of the aspartic and/or glutamic acids of A and P; the group originating from the dialdehyde structure obtained after oxidation of the carbohydrate structure of P or optionally of A, with periodic acid; and the -NH- group originating from one of the terminal amines of A and P or from one of the amines in the epsilon position of one of the lysine residues;
- Y and Y' represent groups covalently bonded to any one of the groups Z and Z' of the proteins A and P;
- E and E' represent inert spacing structures; and
- n represents zero or 1.

**3.** An immunotoxin having the statistical following formula:

$P'(W'-A')_m$

in which:
W' is as defined in claim 2; m varies from 0.3 to 12; P' represents the radical of a protein P which is an antibody or antibody fragment, as such or appropriately chemically modified, from which one or more of its own functions have been removed and in which other functional groups are optionally blocked; A' represents the radical of a protein A, which is trichokirin as defined in claim 1.

**4.** An immunotoxin according to any one of claims 1 to 3, characterized in that the protein A is the modified trichokirin represented by the formula:

$$TK^{o}-(CO-\underset{\displaystyle CH_2COOH}{\underset{|}{CH}}-S-Q)_p$$

in which:
$TK^o$ represents the radical of the trichokirin, Q represents hydrogen or an acetyl group and p varies from 0.2 to 3.

**5.** An immunotoxin as claimed in claim 3, having the following statistical formula:

$$P'-(NH-CO-CH_2CH_2-S-S-\underset{\displaystyle CH_2COOH}{\underset{|}{CH}}-CO-NH-TK')_m$$

in which:
P' and m are as defined in claim 3, and TK' is the radical of the trichokirin as such, from which the amino functions have been removed.

**6.** An immunotoxin according to claim 3, having the following statistical formula:

$P'-(NH-CO-CH_2CH_2-S-S-CH_2CH_2-CO-O-TK'')_m$

in which:
P' and m are as defined in claim 3 and TK" is the radical of the trichokirin as such, from which the phenolic hydroxyl functions have been removed.

**7.** Process for the preparation of an immunotoxin having the following statistical formula:

P'-W-A'

in which:
P' represents the radical of a protein P which is an antibody or antibody fragment, as such or appropriately chemically modified, from which one or more of its own functions have been removed and in which other functional groups are optionally blocked, A' represents the radical of a protein A which is trichokirin, as such or appropriately chemically modified, from which one or more of its own functions have been removed and in which other functional groups are optionally blocked, and W represents a divalent covalent structure containing at least one thioether group or one disulfide group, in which either one of the sulfur atoms is that of the cysteines of P or A, or it is bonded to the own functions of P and/or A by spacing structures carrying a functional group bonded to the own functions of P and/or A, characterized in that a protein $P_1$, which is arbitrarily either the optionally modified trichokirin or an antibody or an antibody fragment, carrying at least one free thiol group attached to said protein $P_1$, directly or via a spacing structure, is reacted, in aqueous solution and at ambient temperature, with a protein P2, different from $P_1$, which is arbitrarily either the optionally modified trichokirin or an antibody or antibody fragment, carrying a group capable of coupling with the free thiol of the protein P1 to form either a thioether or a disulfide bond.

**8.** Process according to claim 8, characterized in that protein derivatives having the statistical formula:

P2" - O - CO - E - G (VI)

in which:
- $P_2$" represents the radical of a protein selected from the group comprising:
  . any antibody or antibody fragment, any immunoglobulin or immunoglobulin fragment or any molecule derived from these molecules by artificial modification of any one of their functional groups;

. trichokirin or any molecule derived from said protein by artificial modification of any one of its functional groups,

the said radical being the protein from which one or more phenolic hydroxyl groups of the tyrosines have been removed;

- the oxygen atom is that belonging to the phenolic hydroxyl groups missing from the radical $P_2$";
- E is as defined in claim 2, and
- G represents a group:

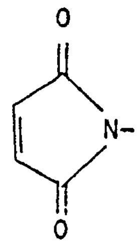

or a -S-S-X group in which X is an activating group.

**9.** Process according to claim 7, characterized in that the protein A is the modified protein having the formula:

$$TK^{o} - (CO - \underset{\substack{| \\ CH_2COOH}}{CH} - S - Q)_p$$

in which:

TK° represents the trichokirin radical, Q represents hydrogen or the acetyl group and p varies from 0.2 and 3.

**10.** Process according to claim 7, for the obtention of an immunotoxin having the statistical formula:

$$P'(NH-CO-CH_2CH_2-S-S-\underset{\substack{| \\ CH_2COOH}}{CH}-CO-NH-TK')_m \qquad (IIIa)$$

in which:

P' and m are as defined in claim 3 and TK' is the radical of the trichokirin as such, from which its amino functions have been removed, characterized in that a protein $P_1$, which is an antibody or an antibody fragment carrying at least one free thiol group bonded to said protein $P_1$ via a spacing structure of formula $-NH-CO-CH_2-CH_2-$, is reacted, in aqueous solution and at ambient temperature, with the mercaptosuccinoyl trichokirin, as protein $P_2$.

**11.** Process according to claim 7, for the obtention of an immunotoxin having the statistical formula:

$P'-(NH-CO-CH_2CH_2-S-S-CH_2CH_2-CO-O-TK'')_m$ (IIIb)

in which:

P' and m are as defined in claim 3, and TK" is the radical of the trichokirin as such, from which its phenolic hydroxyl functions have been removed, characterized in that a protein $P_1$, which is an antibody or an antibody fragment carrying at least one free thiol derivative bonded to said protein $P_1$ via a spacing structure of formula $-NH-CO-CH_2-CH_2-$, is reacted in aqueous solution and at ambient temperature, with the (pyridyl-2 disulfanyl)-3 propionate of trichokirin as protein $P_2$.

**12.** Protein derivative having the statistical formula:

P2" - O - CO - E - G (VI)

in which:

- $P_2$", represents the radical of the trichokirin or any molecule derived from said protein by artificial modification of any one of its functional groups, the said radical being the protein from which one or more phenolic hydroxyl groups of the tyrosines have been removed;
- the oxygen atom is that belonging to the phenolic hydroxyl groups missing from the radical $P_2$";
- E is as defined in claim 2, and
- G represents a group

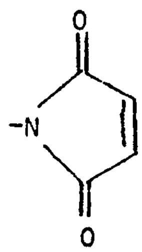

or a -S-S-X group in which X is an activating group.

**13.** Process for the obtention of protein derivatives according to claim 12, characterized in that a product of formula: $P_2$"-OH, in which $P_2$" is as above defined and the hydroxyl group is the phenolic hydroxyl missing from the tyrosines of the radical $P_2$", with a compound of formula (VII) below:

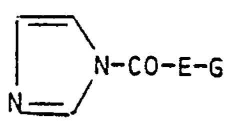

(VII)

at a temperature of 10 to 40° C, in an aqueous solvent optionally containing a water-miscible organic solvent, such as for example an ether solvent like dioxane or tetrahydrofuran.

**14.** Pharmaceutical compositions containing an immunotoxin as claimed in any one of claims 1 to 6, as the active principle.

**Claims for the following Contracting State : ES**

**1.** Process for the obtention of an immunotoxin having the following statistical formula:

P'-W-A'

in which P' represents the radical of a protein P, which is an antibody or antibody fragment, as such or appropriately chemically modified, from which one or more of its own functions have been removed and in which other functional groups are optionally blocked, A' represents the radical of a protein A which is trichokirin, as such or appropriately chemically modified, from which one or more of its own functions have been removed and in which other functional groups are optionally blocked, and W represents a divalent covalent structure containing at least one thioether group or one disulfide group in which either one of the sulfur atoms is that of the cysteines of P or A, or it is bonded to the own functions of P and/or A by spacing structures carrying a functional group bonded to the own functions of P and/or A,

characterized in that a protein $P_1$, which is arbitrarily either the optionally modified trichokirin or an antibody or an antibody fragment, carrying at least one free thiol group attached to said protein $P_1$, directly or via a spacing structure, is reacted, in aqueous solution and at ambient temperature, with a protein P2, different from $P_1$, which is arbitrarily either the optionally modified trichokirin or an antibody or antibody fragment, carrying a group capable of coupling with the free thiol of the protein P1 to form

either a thioether or a disulfide bond, the trichokirin being a glycoprotein:

- having a molecular weight of 28.000 ± 3.000 determined by electrophoresis on polyacrylamide gel in the presence of SDS (sodium dodecyl-sulfate);
- with an isoelectric point ≧ 9;
- with a content of neutral sugars of 1.1 to 1.5% by weight, including 0.3 to 1.2% of mannose;
- having the following amino acid composition, expressed as the number of residues per mol of protein ± 20%:

| | | | |
|---|---|---|---|
| Lys : 17.3 ; | His : 1.1 ; | Arg : 6.7 ; | Asx : 22.8 ; |
| Thr : 18.9 ; | Ser : 23.5 ; | Glx : 21.6 ; | Pro : 8.0 ; |
| Gly : 16.0 ; | Ala : 21.1 ; | 1/2 Cys : 1.9 ; | |
| Val : 12.5 ; | Met : 3.05 ; | Ile : 15.8 ; | Leu : 24.3 ; |
| Tyr : 12.1 ; | Phe : 10.1 ; | Trp : n.d.. | |

where Asx is the aspartic acid and asparagine residues together, Glx is the glutamic acid and glutamine residues together, 1/2 Cys is determined as being the cysteic residues of the native protein in the form of the cysteic acid determined during the analysis and n.d. means "not determined"; and

- it has the following terminal amino sequence:

1 10
Asp-Val-Ser-Phe-Ser-Leu-Ser-Gly-Gly-Gly-
16
Thr-Ala-Ser-Tyr-Glu-Lys

2. Process for the obtention of an immunotoxin having the following statistical formula:

P'-W'-A' II

in which:
P' and A' are as defined in claim 1 and W' represents a covalent structure chosen from:
a) a group of formula:

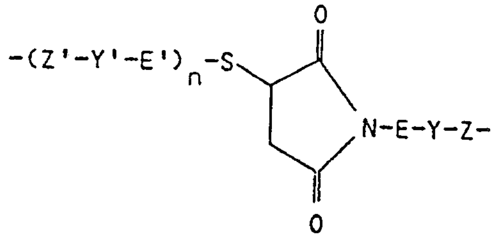

b) a group of formula:

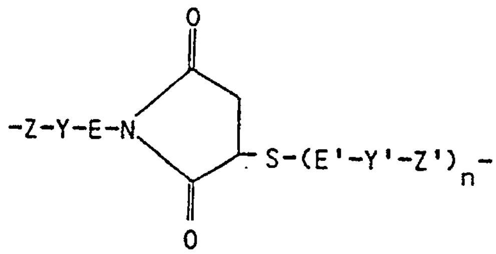

c) a group of formula:

-Z'-Y'-E'-S-S-(E-Y-Z)$_n$-

d) a group of formula:

-S-S-(E-Y-Z)$_n$-

in which:

- Z and Z', which are identical or different, represent the own functions of the proteins A and P, chosen from the oxygen atom originating from the hydroxyl of one of the tyrosine residues; the carbonyl group originating from one of the terminal carboxyls or the free carboxyls of the aspartic and/or glutamic acids of A and P; the group originating from the dialdehyde structure obtained after oxidation of the carbohydrate structure of P or optionally of A, with periodic acid; and the -NH- group originating from one of the terminal amines of A and P or from one of the amines in the epsilon position of one of the lysine residues;
- Y and Y' represent groups covalently bonded to any one of the groups Z and Z' of the proteins A and P;
- E and E' represent inert spacing structures; and
- n represents zero or 1, characterized in that a protein $P_1$, which is arbitrarily either the optionally modified trichokirin or an antibody fragment, carrying at least one free thiol group attached to said protein $P_1$, directly or via a spacing structure, is reacted, in aqueous solution and at ambient temperature, with a protein P2, different from $P_1$, which is arbitrarily either the optionally modified trichokirin or an antibody or antibody fragment, carrying a group capable of coupling with the free thiol of the protein P1 to form either a thioether or a disulfide bond.

**3.** Process for the obtention of an immunotoxin having the following statistical formula:

P'(W' - A')$_m$

in which:
W' is as defined in claim 2; m varies from 0.3 to 12; P' and A are as defined in claim 1, characterized in that a protein $P_1$, which is arbitrarily either the optionally modified trichokirin or an antibody or an antibody fragment, carrying at least one free thiol group attached to said protein $P_1$, directly or via a spacing structure, is reacted, in aqeuous solution and at ambient temperature, with a protein P2, different from $P_1$, which is arbitrarily either the optionally modified trichokirin or an antibody or antibody fragment, carrying a group capable of coupling with the free thiol of the protein P1 to form either a thioether or a disulfide bond.

**4.** Process according to claim 3, for the obtention of an immunotoxin having the following statistical formula:

$$P'-(NH-CO-CH_2CH_2-S-S-\underset{\substack{|\\ CH_2COOH}}{CH}-CO-NH-TK')_m \qquad (IIIa)$$

in which:
P' and m are as defined in claim 3 and TK' is the radical of the trichokirin as such, from which its amino functions have been removed, characterized in that a protein $P_1$, which is an antibody or an antibody fragment carrying at least one free thiol group bonded to said protein $P_1$ via a spacing structure of formula $-NH-CO-CH_2-CH_2-$, is reacted, in aqueous solution and at ambient temperature, with the mercaptosuccinoyl trichokirin, as protein $P_2$.

**5.** Process according to claim 3, for the obtention of an immunotoxin having the following statistical formula:

$P'-(NH-CO-CH_2CH_2-S-S-CH_2CH_2-CO-O-TK'')_m$ (IIIb)

in which:
P' and m are as defined in claim 3, and TK" is the radical of the trichokirin as such, from which its phenolic hydroxyl functions have been removed, characterized in that a protein $P_1$, which is an antibody or an antibody fragment carrying at least one free thiol derivative bonded to said protein $P_1$ via a spacing structure of formula $-NH-CO-CH_2-CH_2-$, is reacted in aqueous solution and at ambient temperature, with the (pyridyl-2 disulfanyl)-3 propionate of trichokirin as protein $P_2$.

**6.** Process according to any one of claims 1 to 3, characterized in that the protein A is the modified protein having the formula:

$$TK^{o} - (CO - \underset{\substack{|\\ CH_2COOH}}{CH} - S - Q)_p$$

in which:
$TK^{o}$ represents the trichokirin radical, Q represents hydrogen or the acetyl group and p varies from 0.2 and 3.

**7.** Process according to any one of claims 1 to 6, characterized in that protein derivatives of the statistical formula:

$P_2'' - O - CO - E - G$ VI

in which:
- $P_2''$, represents the radical of the trichokirin or any molecule derived from said protein by artificial modification of any one of its functional groups, the said radical being the protein from which one or more phenolic hydroxyl groups of the tyrosines have been removed;
- the oxygen atom is that belonging to the phenolic hydroxyl groups missing from the radical $P_2''$;
- E is as defined in claim 2, and
- G represents a group

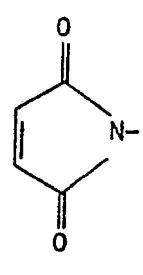

or a -S-S-X group in which X is an activating group, are used as protein $P_1$ or $P_2$.

**8.** Process for the obtention of protein derivatives of formula:

P2" - O - CO - E - G (VI)

in which:
E and G are as defined in claim 7,

- $P_2$" represents the radical of a protein selected from the group comprising:
  . any antibody or antibody fragment, any immunoglobulin or immunoglobulin fragment or any molecule derived from these molecules by artificial modification of any one of their functional groups; said radical being the protein free from one or more phenolic hydroxyl groups of the tyrosines, characterized in that a product of formula: $P_2$"-OH, in which $P_2$" is as above defined and the hydroxyl group is the phenolic hydroxyl missing from the tyrosines of the radical $P_2$", with a compound of formula (VII) below:

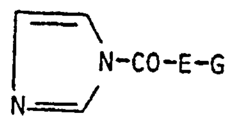 (VII)

at a temperature of 10 to 40° C, in an aqueous solvent optionally containing a water-miscible organic solvent, such as for example an ether solvent like dioxane or tetrahydrofuran.

**Claims for the following Contracting State : GR**

1. An immunotoxin formed by the coupling of an antibody with trichokirin, having the following statistical formula:

P'-W-A'

in which P' represents the radical of a protein P, which is an antibody or antibody fragment, as such or appropriately chemically modified, from which one or more of its own functions have been removed and in which other functional groups are optionally blocked, A' represents the radical of a protein A which is trichokirin, as such or appropriately chemically modified, from which one or more of its own functions have been removed and in which other functional groups are optionally blocked, and W represents a divalent covalent structure containing at least one thioether group or one disulfide group in which either one of the sulfur atoms is that of the cysteines of P or A, or it is bonded to the own functions of P and/or A by spacing structures carrying a functional group bonded to the own functions of P and/or A, the trichokirin being a glycoprotein:

- having a molecular weight of 28,000 ± 3 000 as determined by electrophoresis on polyacrylamide gel in the presence of SDS (sodium dodecyl-sulfate);
- with an isoelectric point ≧ 9;
- with a content of neutral sugars of 1.1 to 1.5% by weight, including 0.3 to 1.2% of mannose;
- having the following amino acid composition, expressed as the number of residues per mol of protein ± 20%:

| | | | | | | | |
|---|---|---|---|---|---|---|---|
| Lys : | 17.3 ; | His : | 1.1 ; | Arg : | 6.7 ; | Asx : | 22.8 ; |
| Thr : | 18.9 ; | Ser : | 23.5 ; | Glx : | 21.6 ; | Pro : | 8.0 ; |
| Gly : | 16.0 ; | Ala : | 21.1 ; | 1/2 Cys : | 1.9 ; | | |
| Val : | 12.5 ; | Met : | 3.05 ; | Ile : | 15.8 ; | Leu : | 24.3 ; |
| Tyr : | 12.1 ; | Phe : | 10.1 ; | Trp : | n.d.. | | |

where Asx is the aspartic acid and asparagine residues together, Glx is the glutamic acid and glutamine residues together, 1/2 Cys is determined as being the cysteic residues of the native protein in the form of the cysteic acid determined during the analysis and n.d. means "not determined"; and

- it has the following terminal amino sequence:

```
1                                   10
Asp-Val-Ser-Phe-Ser-Leu-Ser-Gly-Gly-Gly-
                        16
Thr-Ala-Ser-Tyr-Glu-Lys
```

2. An immunotoxin formed by coupling an antibody with the trichokirin having the following statistical formula:

P'-W'-A'    II

in which:
P' and A' are as defined in claim 1 and W' represents a covalent structure chosen from:

a) a group of formula:

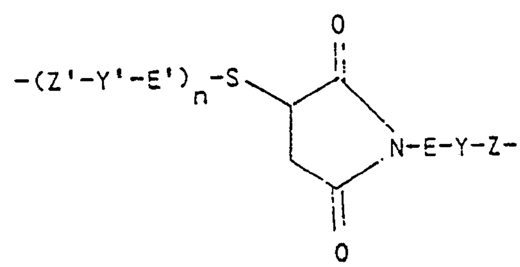

b) a group of formula:

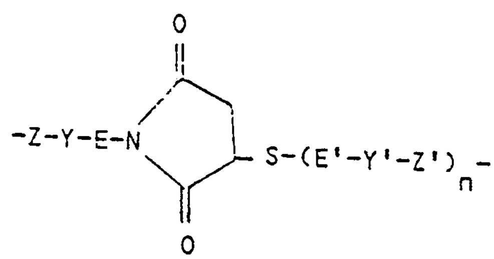

c) a group of formula:

-Z'-Y'-E'-S-S-$(E\text{-}Y\text{-}Z)_n$-

d) a group of formula:

- S-S-$(E\text{-}Y\text{-}Z)_n$-

in which:

- Z and Z', which are identical or different, represent the own functions of the proteins A and P, chosen from the oxygen atom originating from the hydroxyl of one of the tyrosine residues; the carbonyl group originating from one of the terminal carboxyls or the free carboxyls of the aspartic and/or glutamic acids of A and P; the group originating from the dialdehyde structure obtained after oxidation of the carbohydrate structure of P or optionally of A, with periodic acid; and the -NH- group originating from one of the terminal amines of A and P or from one of the amines in the epsilon position of one of the lysine residues;

- Y and Y' represent groups covalently bonded to any one of the groups Z and Z' of the proteins A and P;
- E and E' represent inert spacing structures; and
- n represents zero or 1.

**3.** An immunotoxin having the statistical following formula:

$P'(W'-A')_m$

in which:
W' is as defined in claim 2; m varies from 0.3 to 12; P' represents the radical of a protein P which is an antibody or antibody fragment, as such or appropriately chemically modified, from which one or more of its own functions have been removed and in which other functional groups are optionally blocked; A' represents the radical of a protein A, which is trichokirin as defined in claim 1.

**4.** An immunotoxin according to any one of claims 1 to 3, characterized in that the protein A is the modified trichokirin represented by the formula:

$$TK^{o} - (CO - \underset{\displaystyle CH_2COOH}{\underset{|}{CH}} - S - Q)_p$$

in which:
$TK^o$ represents the radical of the trichokirin, Q represents hydrogen or an acetyl group and p varies from 0.2 to 3.

**5.** An immunotoxin as claimed in claim 3, having the following statistical formula:

$$P'-(NH-CO-CH_2CH_2-S-S-\underset{\displaystyle CH_2COOH}{\underset{|}{CH}}-CO-NH-TK')_m$$

in which:
P' and m are as defined in claim 3, and TK' is the radical of the trichokirin as such, from which the amino functions have been removed.

**6.** An immunotoxin according to claim 3, having the following statistical formula:

$P'-(NH-CO-CH_2CH_2-S-S-CH_2CH_2-CO-O-TK'')_m$

in which:
P' and m are as defined in claim 3 and TK" is the radical of the trichokirin as such, from which the phenolic hydroxyl functions have been removed.

**7.** Process for the preparation of an immunotoxin having the following statistical formula:

P'-W-A'

in which:
P' represents the radical of a protein P which is an antibody or antibody fragment, as such or appropriately chemically modified, from which one or more of its own functions have been removed and in which other functional groups are optionally blocked, A' represents the radical of a protein A which is trichokirin, as such or appropriately chemically modified, from which one or more of its own functions have been removed and in which other functional groups are optionally blocked, and W represents a divalent covalent structure containing at least one thioether group or one disulfide group, in which either one of the sulfur atoms is that of the cysteines of P or A, or it is bonded to the own

functions of P and/or A by spacing structures carrying a functional group bonded to the own functions of P and/or A, characterized in that a protein $P_1$, which is arbitrarily either the optionally modified trichokirin or an antibody or an antibody fragment, carrying at least one free thiol group attached to said protein $P_1$, directly or via a spacing structure, is reacted, in aqueous solution and at ambient temperature, with a protein P2, different from $P_1$, which is arbitrarily either the optionally modified trichokirin or an antibody or antibody fragment, carrying a group capable of coupling with the free thiol of the protein P1 to form either a thioether or a disulfide bond.

**8.** Process according to claim 8, characterized in that protein derivatives having the statistical formula:

P2" - O - CO - E - G (VI)

in which:

- $P_2$" represents the radical of a protein selected from the group comprising:
  . any antibody or antibody fragment, any immunoglobulin or immunoglobulin fragment or any molecule derived from these molecules by artificial modification of any one of their functional groups;
  . trichokirin or any molecule derived from said protein by artificial modification of any one of its functional groups,

  the said radical being the protein from which one or more phenolic hydroxyl groups of the tyrosines have been removed;
- the oxygen atom is that belonging to the phenolic hydroxyl groups missing from the radical $P_2$";
- E is as defined in claim 2, and
- G represents a group:

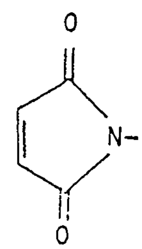

or a -S-S-X group in which X is an activating group.

**9.** Process according to claim 7, characterized in that the protein A is the modified protein having the formula:

$$TK^{o} - (CO - \underset{\displaystyle CH_2COOH}{CH} - S - Q)_p$$

in which:

$TK^o$ represents the trichokirin radical, Q represents hydrogen or the acetyl group and p varies from 0.2 and 3.

**10.** Process according to claim 7, for the obtention of an immunotoxin having the statistical formula:

$$P'(NH-CO-CH_2CH_2-S-S-\underset{\displaystyle CH_2COOH}{CH}-CO-NH-TK')_m \qquad (IIIa)$$

in which:

P' and m are as defined in claim 3 and TK' is the radical of the trichokirin as such, from which its

amino functions have been removed, characterized in that a protein $P_1$, which is an antibody or an antibody fragment carrying at least one free thiol group bonded to said protein $P_1$ via a spacing structure of formula $-NH-CO-CH_2-CH_2-$, is reacted, in aqueous solution and at ambient temperature, with the mercaptosuccinoyl trichokirin, as protein $P_2$.

**11.** Process according to claim 7, for the obtention of an immunotoxin having the statistical formula:

$P'-(NH-CO-CH_2CH_2-S-S-CH_2CH_2-CO-O-TK")_m$ (IIIb)

in which:
P' and m are as defined in claim 3, and TK" is the radical of the trichokirin as such, from which its phenolic hydroxyl functions have been removed, characterized in that a protein $P_1$, which is an antibody or an antibody fragment carrying at least one free thiol derivative bonded to said protein $P_1$ via a spacing structure of formula $-NH-CO-CH_2-CH_2-$, is reacted in aqueous solution and at ambient temperature, with the (pyridyl-2 disulfanyl)-3 propionate of trichokirin as protein $P_2$.

**12.** Protein derivative having the statistical formula:

P2" - O - CO - E - G (VI)

in which:
- $P_2$", represents the radical of the trichokirin or any molecule derived from said protein by artificial modification of any one of its functional groups, the said radical being the protein from which one or more phenolic hydroxyl groups of the tyrosines have been removed;
- the oxygen atom is that belonging to the phenolic hydroxyl groups missing from the radical $P_2$";
- E is as defined in claim 2, and
- G represents a group

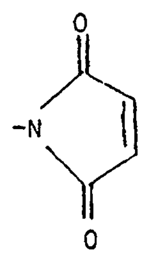

or a -S-S-X group in which X is an activating group.

**13.** Process for the obtention of protein derivatives according to claim 12, characterized in that a product of formula: $P_2$"-OH, in which $P_2$" is as above defined and the hydroxyl group is the phenolic hydroxyl missing from the tyrosines of the radical $P_2$", with a compound of formula (VII) below:

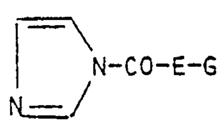

(VII)

at a temperature of 10 to 40° C, in an aqueous solvent optionally containing a water-miscible organic solvent, such as for example an ether solvent like dioxane or tetrahydrofuran.

**Patentansprüche**

**Patentansprüche für folgende Vertragsstaaten : AT, BE, CH, DE, FR, GB, IT, LI, LU, NL, SE**

**1.** Immunotoxin, welches durch Kopplung eines Antikörpers mit Trichokirin gebildet ist und die folgende Analysenformel hat:

P' - W - A'

worin P' den Rest eines Proteins P darstellt, welches ein Antikörper oder ein Antikörperfragment als solches oder zweckmäßig chemisch modifiziert ist, dem eine oder mehrere eigentliche Funktionen entzogen sind, und bei dem andere funktionelle Gruppen gegebenenfalls blockiert sind;
A' den Rest des Proteins A darstellt, welches Trichokirin als solches oder zweckmäßig chemisch modifiziert ist, dem eine oder mehrere eigentliche Funktionen entzogen sind, und bei dem andere funktionelle Gruppen gegebenenfalls blockiert sind; und
W eine bivalente kovalente Struktur darstellt, die mindestens eine Thioethergruppe oder eine Disulfidgruppe enthält, bei der eines der Schwefelatome entweder ausgewählt ist aus jenen der Cysteine von P oder A oder über eine an die eigentlichen Funktionen von P und A gebundene funktionelle Gruppe tragende Raumstrukturen an die eigentlichen Funktionen von P und/oder A gebunden ist, wobei Trichokirin ein Glykoprotein ist

- mit einem Molekulargewicht von 28.000 ± 3.000, bestimmt durch Polyacrylamidgel-Elektrophorese in Gegenwart von SDS (Natriumdodecylsulfat),
- mit einem isoelektrischen Punkt von ≧ 9,
- mit einem Gehalt an neutralen Zuckern von 1,1 bis 1,5 Gew. %, davon 0,3 bis 1,2 % Mannose,
- mit einer Aminosäurenzusammensetzung: Anzahl an Resten pro Mol Protein ± 20 %:

| | | | |
|---|---|---|---|
| Lys : 17,3 ; | His : 1,1 ; | Arg : 6,7 ; | Asx : 22,8 ; |
| Thr : 18,9 ; | Ser : 23,5 ; | Glx : 21,6 ; | Pro : 8,0 ; |
| Gly : 16,0 ; | Ala : 21,1 ; | 1/2 Cys : 1,9 ; | |
| Val : 12,5 ; | Met : 3,05 ; | Ile : 15,8 ; | Leu : 24,3 ; |
| Tyr : 12,1 ; | Phe : 10,1 ; | Trp : n.d.; | |

sodaß Asx die Gesamtheit der Asparaginsäure- und Asparaginreste ist,
Glx die Gesamtheit der Glutaminsäure- und Glutaminreste ist,
1/2 Cys der Cysteinrest des nativen Proteins in Form von Cysteinsäure ist, bestimmt bei der Analyse,
und n.d. für "nicht bestimmt" steht

- mit einer terminalen Aminosequenz:

```
1                                  10
Asp-Val-Ser-Phe-Ser-Leu-Ser-Gly-Gly-Gly-
                             16
Thr-Ala-Ser-Tyr-Glu-Lys
```

**2.** Immunotoxin, welches durch Kopplung eines Antikörpers mit Trichokirin gebildet ist und die folgende Analysenformel hat:

P' - W' - A' (II)

worin P' und A' wie in Anspruch 1 definiert sind und W' eine kovalente Struktur darstellt, ausgewählt aus
(a) einer Gruppe der Formel

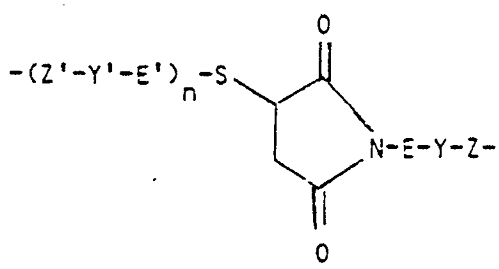

(b) einer Gruppe der Formel

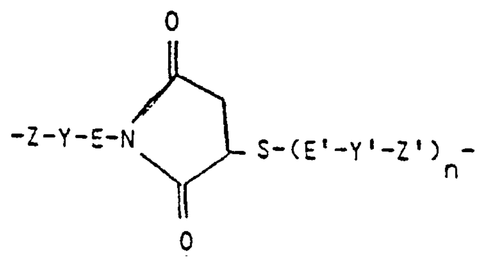

(c) einer Gruppe der Formel

$-Z'-Y'-E'-S-S-(E-Y-Z)_n-$

(d) einer Gruppe der Formel

$-S-S-(E-Y-Z)_n-$

worin:

- Z und Z', gleich oder verschieden, die eigentlichen Funktionen der Proteine A und P darstellen, ausgewählt aus dem vom Hydroxyl eines der Tyrosinreste kommenden Sauerstoffatom; der von einem der terminalen Carboxyle oder der freien Carboxyle der Asparagin- und/oder Glutaminsäure von A und P kommenden Carbonylgruppe; der von der nach Oxidation der Kohlenhydratstruktur von P oder gegebenenfalls von A mit Perjodsäure erhaltenen Dialdehydstruktur kommenden Gruppe; und der von einem der terminalen Amine von A und P oder einem der Amine in Position Epsilon eines der Lysinreste kommenden Gruppe -NH-;
- Y und Y' Gruppen darstellen, die eine kovalente Bindung mit irgendeiner der Funktionen Z und Z' der Proteine A und P eingegangen sind;
- E und E' inerte Raumstrukturen darstellen;
- n für Null oder 1 steht.

**3.** Immunotoxin mit der folgenden Analysenformel

$P'-(W' - A')_m$

worin
W' wie in Anspruch 2 definiert ist; m von 0,3 bis 12 variiert; P' den Rest eines Proteins P darstellt, welches ein Antikörper oder ein Antikörperfragment als solches oder zweckmäßig chemisch modifiziert ist, dem eine oder mehrere eigentliche Funktionen entzogen sind, und bei dem die anderen funktionellen Gruppen gegebenenfalls blockiert sind; A' den Rest des Proteins A darstellt, welches Trichokirin ist, wie in Anspruch 1 definiert.

**4.** Immunotoxin nach einem der Ansprüche 1 bis 3, dadurch gekennzeichnet, daß das Protein A modifiziertes Trichokirin ist, dargestellt durch die Formel

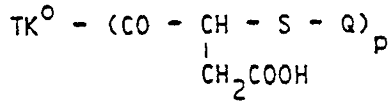

worin
TK° den Rest von Trichokirin darstellt, Q Wasserstoff oder die Gruppe Acetyl bedeutet und p zwischen 0,2 und 3 variiert.

5. Immunotoxin nach Anspruch 3 mit der folgenden Analysenformel

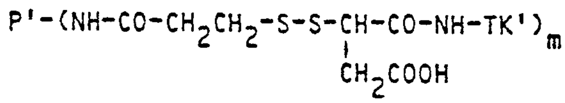

worin
P' und m wie in Anspruch 3 definiert sind und TK' der Rest von Trichokirin als solches ohne Aminofunktionen ist.

6. Immunotoxin nach Anspruch 3 mit der folgenden Analysenformel

$P'\text{-}(NH\text{-}CO\text{-}CH_2CH_2\text{-}S\text{-}S\text{-}CH_2CH_2\text{-}CO\text{-}O\text{-}TK'')_m$

worin
P' und m wie in Anspruch 3 definiert sind und TK" der Rest von Trichokirin als solches ohne phenolische Hydroxylfunktionen ist.

7. Verfahren zur Herstellung eines Immunotoxins mit der folgenden Analysenformel

P' - W - A'

worin
P' den Rest eines Proteins P darstellt, welches ein Antikörper oder ein Antikörperfragment als solches oder zweckmäßig chemisch modifiziert ist, dem eine oder mehrere eigentliche Funktionen entzogen sind, und bei dem die anderen funktionellen Gruppen gegebenenfalls blockiert sind; A' den Rest des Proteins A darstellt, welches Trichokirin als solches oder zweckmäßig chemisch modifiziert ist, dem eine oder mehrere eigentliche Funktionen entzogen sind, und bei dem die anderen funktionellen Gruppen gegebenenfalls blockiert sind; und W eine bivalente kovalente Struktur darstellt, die mindestens eine Thioethergruppe oder eine Disulfidgruppe enthält, bei der eines der Schwefelatome entweder ausgewählt ist aus jenen der Cysteine von P oder A oder über eine an die eigentlichen Funktionen von P und/oder von A gebundene funktionelle Gruppe tragende Raumstrukturen an die eigentlichen Funktionen von P und/oder A gebunden ist; dadurch gekennzeichnet, daß man ein Protein P1, welches ohne Unterschied entweder Trichokirin, gegebenenfalls modifiziert, oder ein Antikörper oder Antikörperfragment und Träger von mindestens einer freien Thiolgruppe ist, welche direkt oder unter Zwischenschaltung einer Raumstruktur am Protein P1 hängt, mit einem Protein P2, welches von P1 unterschiedlich und ohne Unterschied entweder Trichokirin, gegebenenfalls modifiziert, oder ein Antikörper oder Antikörperfragment und Träger einer mit dem freien Thiol des Proteins P1 kopplungsfähigen Gruppe zur Bildung einer Thioether- oder Disulfidbindung in wässeriger Lösung und bei Umgebungstemperatur zur Umsetzung bringt.

8. Verfahren nach Anspruch 7, dadurch gekennzeichnet, daß man als Protein P1 oder P2 ein Proteinderivat verwendet, dargestellt durch die Analysenformel

P2" - O - CO - E - G (VI)

worin
- P2" den Rest eines Proteins, ausgewählt aus:

. jedem Antikörper oder Antikörperfragment oder jedem Immunglobulin oder Immunglobulinfragment oder jedem Molekül, das von den vorhergehenden durch künstliche Modifikation irgendeiner ihrer funktionellen Gruppen stammt,

. Trichokirin oder jedem Molekül, das von diesem Protein durch künstliche Modifikation irgendeiner seiner funktionellen Gruppen stammt,

darstellt, wobei der Rest das Protein ist, dem eine oder mehrere phenolische Hydroxylgruppen der Tyrosine entzogen sind;

- das Sauerstoffatom jenes der phenolischen Hydroxylgruppen ist, die im Rest P2" fehlen;
- E wie in Anspruch 2 definiert ist und
- G für eine Gruppe

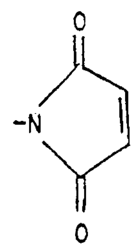

oder eine Gruppe -S-S-X, worin X eine Aktivatorgruppe ist, steht.

**9.** Verfahren nach Anspruch 7, dadurch gekennzeichnet, daß das Protein A modifiziertes Trichokirin der Formel

$$TK^{o}-(CO-\underset{\substack{|\\ CH_2COOH}}{CH}-S-Q)_p$$

ist, worin

TK° den Rest von Trichokirin darstellt, Q für Wasserstoff oder die Gruppe Acetyl steht und p zwischen 0,2 und 3 variiert.

**10.** Verfahren nach Anspruch 7 zur Herstellung eines Immunotoxins mit der Analysenformel

$$P'(NH-CO-CH_2CH_2-S-S-\underset{\substack{|\\ CH_2COOH}}{CH}-CO-NH-TK')_m \qquad (IIIa)$$

worin P' und m wie in Anspruch 3 definiert sind und TK' der Rest von Trichokirin als solches ohne Aminofunktionen, ist, dadurch gekennzeichnet, daß es darin besteht, daß ein Protein P1, welches ein Antikörper oder ein Antikörperfragment und Träger von mindestens einer unter Zwischenschaltung einer Raumstruktur der Formel $-NH-CO-CH_2-CH_2-$ an das Protein P1 gebundenen freien Thiolgruppe ist, mit Mercaptosuccinoyltrichokirin als Protein P2 in wässeriger Lösung und bei Umgebungstemperatur zur Umsetzung gebracht wird.

**11.** Verfahren nach Anspruch 7 zur Herstellung eines Immunotoxins mit der Analysenformel

$P'-(NH-CO-CH_2CH_2-S-S-CH_2CH_2-CO-O-TK")_m$ (IIIb)

worin

P' und m wie in Anspurch 3 definiert sind und TK" der Rest von Trichokirin als solches, ohne phenolische Hydroxylfunktionen ist, dadurch gekennzeichnet, daß es darin besteht, daß ein Protein P1, welches ein Antikörper oder ein Antikörperfragment und Träger von mindestens einer unter Zwischenschaltung einer Raumstruktur der Formel $-NH-CO-CH_2-CH_2-$ an das Protein P1 gebundenen freien Thiolgruppe ist, in wässeriger Lösung und bei Umgebungstemperatur mit Trichokirin-3-(2-Pyridyldisulfanyl)-propionat als Protein P2 zur Umsetzung gebracht wird.

**12.** Proteinderivate der Analysenformel

P2" - O - CO - E - G (VI)

worin

- P2" den Rest von Trichokirin oder jedem Molekül, das von diesem Protein durch künstliche Modifikation irgendeiner seiner funktionellen Gruppen stammt, darstellt;
  wobei der Rest das Protein ist, dem eine oder mehrere phenolische Hydroxylgruppen der Tyrosine entzogen sind;
- das Sauerstoffatom jenes der phenolischen Hydroxylgruppen ist, die im Rest P2" fehlen;
- E wie in Anspruch 2 definiert ist und
- G für eine Gruppe

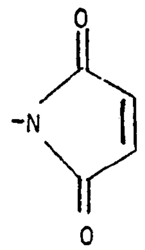

oder eine Gruppe -S-S-X, worin X eine Aktivatorgruppe ist, steht.

**13.** Verfahren zur Herstellung der Proteinderivate nach Anspruch 12, dadurch gekennzeichnet, daß es darin besteht, daß ein Produkt der Formel P2"-OH, worin P2" wie oben definiert und wobei die Hydroxylgruppe das in den Tyrosinen des Restes P2" fehlende phenolische Hydroxyl ist, mit einer Verbindung der nachstehenden Formel (VII)

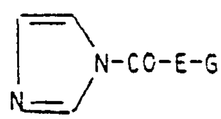

(VII)

bei einer Temperatur von 10 bis 40° C in einem wässerigen Lösungsmittel, das gegebenenfalls ein mit Wasser mischbares organisches Lösungsmittel, wie beispielsweise ein etherisches Lösungsmittel, wie Dioxan oder Tetrahydrofuran, enthält, zur Umsetzung gebracht wird.

**14.** Pharmazeutische Zusammensetzungen, die als Wirkstoff ein Immunotoxin nach einem der Ansprüche 1 bis 6 enthalten.

**Patentansprüche für folgenden Vertragsstaat : ES**

**1.** Verfahren zur Herstellung eines Immunotoxins mit der folgenden Analysenformel

P' - W - A' (I)

worin

P' den Rest eines Proteins P darstellt, welches ein Antikörper oder ein Antikörperfragment als solches oder zweckmäßig chemisch modifiziert ist, dem eine oder mehrere eigentliche Funktionen entzogen sind, und bei dem die anderen funktionellen Gruppen gegebenenfalls blockiert sind; A' den Rest des Proteins A darstellt, welches Trichokirin als solches oder zweckmäßig chemisch modifiziert ist, dem eine oder mehrere eigentliche Funktionen entzogen sind, und bei dem die anderen funktionellen Gruppen gegebenenfalls blockiert sind; und W eine bivalente kovalente Struktur darstellt, die mindestens eine Thioethergruppe oder eine Disulfidgruppe enthält, bei der eines der Schwefelatome entweder ausgewählt ist aus jenen der Cysteine von P oder A oder über eine an die eigentlichen

Funktionen von P und/oder von A gebundene funktionelle Gruppe tragende Raumstrukturen an die eigentlichen Funktionen von P und/oder A gebunden ist; dadurch gekennzeichnet, daß man ein Protein P1, welches ohne Unterschied entweder Trichokirin, gegebenenfalls modifiziert, oder ein Antikörper oder Antikörperfragment und Träger von mindestens einer freien Thiolgruppe ist, welche direkt oder unter Zwischenschaltung einer Raumstruktur am Protein P1 hängt, mit einem Protein P2, welches von P1 unterschiedlich und ohne Unterschied entweder Trichokirin, gegebenenfalls modifiziert, oder ein Antikörper oder Antikörperfragment und Träger einer mit dem freien Thiol des Proteins P1 kopplungsfähigen Gruppe zur Bildung einer Thioether- oder Disulfidbindung in wässeriger Lösung und bei Umgebungstemperatur zur Umsetzung bringt, wobei Trichokirin ein Glykoprotein ist

- mit einem Molekulargewicht von 28.000 ± 3.000, bestimmt durch Polyacrylamidgel-Elektrophorese in Gegenwart von SDS (Natriumdodecylsulfat),
- mit einem isoelektrischen Punkt von ≧ 9,
- mit einem Gehalt an neutralen Zuckern von 1,1 bis 1,5 Gew. %, davon 0,3 bis 1,2 % Mannose,
- mit einer Aminosäurenzusammensetzung: Anzahl an Resten pro Mol Protein ± 20 %:

| | | | |
|---|---|---|---|
| Lys : 17,3 ; | His : 1,1 ; | Arg : 6,7 ; | Asx : 22,8 ; |
| Thr : 18,9 ; | Ser : 23,5 ; | Glx : 21,6 ; | Pro : 8,0 ; |
| Gly : 16,0 ; | Ala : 21,1 ; | 1/2 Cys : 1,9 ; | |
| Val : 12,5 ; | Met : 3,05 ; | Ile : 15,8 ; | Leu : 24,3 ; |
| Tyr : 12,1 ; | Phe : 10,1 ; | Trp : n.d.; | |

sodaß Asx die Gesamtheit der Asparaginsäure- und Asparaginreste ist,
Glx die Gesamtheit der Glutaminsäure- und Glutaminreste ist,
1/2 Cys der Cysteinrest des nativen Proteins in Form von Cysteinsäure ist, bestimmt bei der Analyse,
und n.d. für "nicht bestimmt" steht

- mit einer terminalen Aminosequenz:

1 10
Asp-Val-Ser-Phe-Ser-Leu-Ser-Gly-Gly-Gly-
16
Thr-Ala-Ser-Tyr-Glu-Lys

2. Verfahren zur Herstellung eines Immunotoxins der folgenden Analysenformel:

P' - W' - A' (II)

worin P' und A' wie in Anspruch 1 definiert sind und W' eine kovalente Struktur darstellt, ausgewählt aus

(a) einer Gruppe der Formel

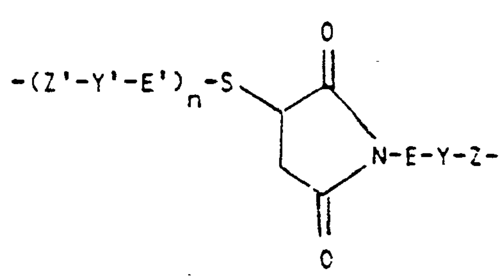

(b) einer Gruppe der Formel

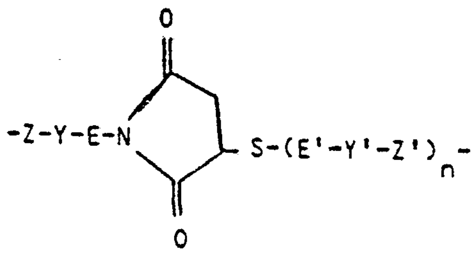

(c) einer Gruppe der Formel

$-Z'-Y'-E'-S-S-(E-Y-Z)_n-$

(d) einer Gruppe der Formel

$-S-S-(E-Y-Z)_n-$

worin:

- Z und Z', gleich oder verschieden, die eigentlichen Funktionen der Proteine A und P darstellen, ausgewählt aus dem vom Hydroxyl eines der Tyrosinreste kommenden Sauerstoffatom; der von einem der terminalen Carboxyle oder der freien Carboxyle der Asparagin- und/oder Glutaminsäure von A und P kommenden Carbonylgruppe; der von der nach Oxidation der Kohlenhydratstruktur von P oder gegebenenfalls von A mit Perjodsäure erhaltenen Dialdehydstruktur kommenden Gruppe; und der von einem der terminalen Amine von A und P oder einem der Amine in Position Epsilon eines der Lysinreste kommenden Gruppe -NH-;
- Y und Y' Gruppen darstellen, die eine kovalente Bindung mit irgendeiner der Funktionen Z und Z' der Proteine A und P eingegangen sind;
- E und E' inerte Raumstrukturen darstellen;
- n für Null oder 1 steht,

dadurch gekennzeichnet, daß man ein Protein P1, welches ohne Unterschied entweder Trichokirin, gegebenenfalls modifiziert, oder ein Antikörper oder Antikörperfragment und Träger von mindestens einer freien Thiolgruppe ist, welche direkt oder unter Zwischenschaltung einer Raumstruktur am Protein P1 hängt, mit einem Protein P2, welches von P1 unterschiedlich und ohne Unterschied entweder Trichokirin, gegebenenfalls modifiziert, oder ein Antikörper oder Antikörperfragment und Träger einer mit dem freien Thiol des Proteins P1 kopplungsfähigen Gruppe zur Bildung einer Thioether- oder Disulfidbindung in wässeriger Lösung und bei Umgebungstemperatur zur Umsetzung bringt.

**3.** Verfahren zur Herstellung eines Immunotoxins mit der folgenden Analysenformel

$P'-(W' - A')_m$

worin

W' wie in Anspruch 2 definiert ist; m von 0,3 bis 12 variiert; P' und A' wie in Anspruch 1 definiert sind,

dadurch gekennzeichnet, daß man ein Protein P1, welches ohne Unterschied entweder Trichokirin, gegebenenfalls modifiziert, oder ein Antikörper oder Antikörperfragment und Träger von mindestens einer freien Thiolgruppe ist, welche direkt oder unter Zwischenschaltung einer Raumstruktur am Protein P1 hängt, mit einem Protein P2, welches von P1 unterschiedlich und ohne Unterschied entweder Trichokirin, gegebenenfalls modifiziert, oder ein Antikörper oder Antikörperfragment und Träger einer mit dem freien Thiol des Proteins P1 kopplungsfähigen Gruppe zur Bildung einer Thioether- oder Disulfidbindung in wässeriger Lösung und bei Umgebungstemperatur zur Umsetzung bringt.

**4.** Verfahren nach Anspruch 3 zur Herstellung eines Immunotoxins mit der folgenden Analysenformel

$$P'(NH-CO-CH_2CH_2-S-S-\underset{CH_2COOH}{CH}-CO-NH-TK')_m \qquad (IIIa)$$

worin P' und m wie in Anspruch 3 definiert sind und TK' der Rest von Trichokirin als solches ohne Aminofunktionen, ist, dadurch gekennzeichnet, daß es darin besteht, daß ein Protein P1, welches ein Antikörper oder ein Antikörperfragment und Träger von mindestens einer direkt oder unter Zwischenschaltung einer Raumstruktur an das Protein P1 gebundenen freien Thiolgruppe ist, mit einem Protein P2, welches Mercaptosuccinoyltrichokirin ist, in wässeriger Lösung und bei Umgebungstemperatur zur Umsetzung gebracht wird.

5. Verfahren nach Anspruch 3 zur Herstellung eines Immunotoxins mit der Analysenformel

$P'-(NH-CO-CH_2CH_2-S-S-CH_2CH_2-CO-O-TK")_m$ (IIIb)

worin
P' und m wie in Anspurch 3 definiert sind und TK" der Rest von Trichokirin als solches, ohne phenolische Hydroxylfunktionen ist, dadurch gekennzeichnet, daß es darin besteht, daß ein Protein P1, welches ein Antikörper oder ein Antikörperfragment und Träger von mindestens einer direkt oder unter Zwischenschaltung einer Raumstruktur an das Protein P1 gebundenen freien Thiolgruppe ist, mit einem Protein P2, welches Trichokirin-3-(2-pyridyldisulfanyl)-propionat ist, in wässeriger Lösung und bei Umgebungstemperatur zur Umsetzung gebracht wird.

6. Verfahren nach einem der Ansprüche 1 bis 3, dadurch gekennzeichnet, daß das Protein A modifiziertes Trichokirin der Formel

$$TK^o - (CO - \underset{CH_2COOH}{CH} - S - Q)_p$$

ist, worin
$TK^o$ den Rest von Trichokirin darstellt, Q für Wasserstoff oder die Gruppe Acetyl steht und p zwischen 0,2 und 3 variiert.

7. Verfahren nach einem der Ansprüche 1 bis 6, dadurch gekennzeichnet, daß man als Protein P1 oder P2 Proteinderivate der Analysenformel verwendet:

P2" - O - CO - E - G (VI)

worin
- P2" den Rest eines Proteins, ausgewählt aus:
  . jedem Antikörper oder Antikörperfragment oder jedem Immunglobulin oder Immunglobulinfragment oder jedem Molekül, das von den vorhergehenden durch künstliche Modifikation irgendeiner ihrer funktionellen Gruppen stammt,
  . Trichokirin oder jedem Molekül, das von diesem Protein durch künstliche Modifikation irgendeiner seiner funktionellen Gruppen stammt,

  darstellt, wobei der Rest das Protein ist, dem eine oder mehrere phenolische Hydroxylgruppen der Tyrosine entzogen sind;
- das Sauerstoffatom jenes der phenolischen Hydroxylgruppen ist, die im Rest P2" fehlen;
- E wie in Anspruch 2 definiert ist und
- G für eine Gruppe

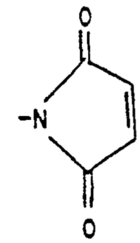

oder eine Gruppe -S-S-X, worin X eine Aktivatorgruppe ist, steht.

8. Verfahren zur Herstellung von Proteinderivaten der Analysenformel:

P2" - O - CO - E - G (VI)

worin
E und G wie in Anspruch 7 definiert sind, P2" den Rest von Trichokirin oder jedem Molekül, das von diesem Protein durch künstliche Modifikation irgendeiner seiner funktionellen Gruppen stammt, darstellt,
wobei der Rest das Protein ist, dem eine oder mehrere phenolische Hydroxylgruppen der Tyrosine entzogen sind;
dadurch gekennzeichnet, daß es darin besteht, daß ein Produkt der Formel P2"-OH, worin P2" wie oben definiert und wobei die Hydroxylgruppe das in den Tyrosinen des Restes P2" fehlende phenolische Hydroxyl ist, mit einer Verbindung der nachstehenden Formel (VII)

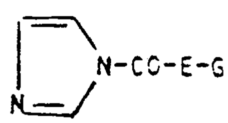

(VII)

worin E und G wie oben definiert sind, bei einer Temperatur von 10 bis 40 °C in einem wässerigen Lösungsmittel, das gegebenenfalls ein mit Wasser mischbares organisches Lösungsmittel, wie beispielsweise ein etherisches Lösungsmittel, wie Dioxan oder Tetrahydrofuran, enthält, zur Umsetzung gebracht wird.

**Patentansprüche für folgenden Vertragsstaat : GR**

1. Immunotoxin, welches durch Kopplung eines Antikörpers mit Trichokirin gebildet ist und die folgende Analysenformel hat:

P' - W - A'

worin P' den Rest eines Proteins P darstellt, welches ein Antikörper oder ein Antikörperfragment als solches oder zweckmäßig chemisch modifiziert ist, dem eine oder mehrere eigentliche Funktionen entzogen sind, und bei dem andere funktionelle Gruppen gegebenenfalls blockiert sind;
A' den Rest des Proteins A darstellt, welches Trichokirin als solches oder zweckmäßig chemisch modifiziert ist, dem eine oder mehrere eigentliche Funktionen entzogen sind, und bei dem andere funktionelle Gruppen gegebenenfalls blockiert sind; und
W eine bivalente kovalente Struktur darstellt, die mindestens eine Thioethergruppe oder eine Disulfidgruppe enthält, bei der eines der Schwefelatome entweder ausgewählt ist aus jenen der Cysteine von P oder A oder über eine an die eigentlichen Funktionen von P und A gebundene funktionelle Gruppe tragende Raumstrukturen an die eigentlichen Funktionen von P und/oder A gebunden ist, wobei Trichokirin ein Glykoprotein ist

- mit einem Molekulargewicht von 28.000 ± 3.000, bestimmt durch Polyacrylamidgel-Elektrophorese in Gegenwart von SDS (Natriumdodecylsulfat),
- mit einem isoelektrischen Punkt von ≧ 9,

- mit einem Gehalt an neutralen Zuckern von 1,1 bis 1,5 Gew. %, davon 0,3 bis 1,2 % Mannose,
- mit einer Aminosäurenzusammensetzung: Anzahl an Resten pro Mol Protein ± 20 %:

| | | | |
|---|---|---|---|
| Lys : 17,3 ; | His : 1,1 ; | Arg : 6,7 ; | Asx : 22,8 ; |
| Thr : 18,9 ; | Ser : 23,5 ; | Glx : 21,6 ; | Pro : 8,0 ; |
| Gly : 16,0 ; | Ala : 21,1 ; | 1/2 Cys : 1,9 ; | |
| Val : 12,5 ; | Met : 3,05 ; | Ile : 15,8 ; | Leu : 24,3 ; |
| Tyr : 12,1 ; | Phe : 10,1 ; | Trp : n.d., | |

sodaß Asx die Gesamtheit der Asparaginsäure- und Asparaginreste ist,
Glx die Gesamtheit der Glutaminsäure- und Glutaminreste ist,
1/2 Cys der Cysteinrest des nativen Proteins in Form von Cysteinsäure ist, bestimmt bei der Analyse,
und n.d. für "nicht bestimmt" steht
- mit einer terminalen Aminosequenz:

1 10
Asp-Val-Ser-Phe-Ser-Leu-Ser-Gly-Gly-Gly-
16
Thr-Ala-Ser-Tyr-Glu-Lys

2. Immunotoxin, welches durch Kopplung eines Antikörpers mit Trichokirin gebildet ist und die folgende Analysenformel hat:

P' - W' - A' (II)

worin P' und A' wie in Anspruch 1 definiert sind und W' eine kovalente Struktur darstellt, ausgewählt aus
(a) einer Gruppe der Formel

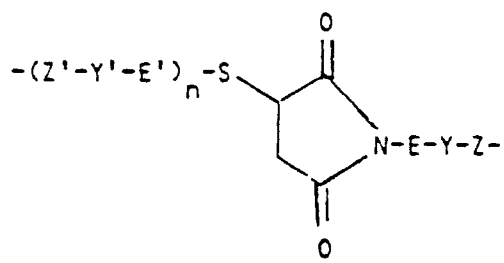

(b) einer Gruppe der Formel

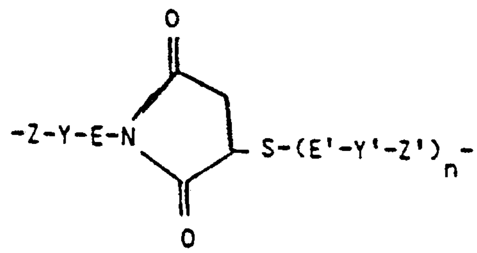

(c) einer Gruppe der Formel

$-Z'-Y'-E'-S-S-(E-Y-Z)_n-$

(d) einer Gruppe der Formel

$-S-S-(E-Y-Z)_n-$

worin:
- Z und Z', gleich oder verschieden, die eigentlichen Funktionen der Proteine A und P darstellen, ausgewählt aus dem vom Hydroxyl eines der Tyrosinreste kommenden Sauerstoffatom; der von einem der terminalen Carboxyle oder der freien Carboxyle der Asparagin- und/oder Glutaminsäure von A und P kommenden Carbonylgruppe; der von der nach Oxidation der Kohlenhydratstruktur von P oder gegebenenfalls von A mit Perjodsäure erhaltenen Dialdehydstruktur kommenden Gruppe; und der von einem der terminalen Amine von A und P oder einem der Amine in Position Epsilon eines der Lysinreste kommenden Gruppe -NH-;
- Y und Y' Gruppen darstellen, die eine kovalente Bindung mit irgendeiner der Funktionen Z und Z' der Proteine A und P eingegangen sind;
- E und E' inerte Raumstrukturen darstellen;
- n für Null oder 1 steht.

**3.** Immunotoxin mit der folgenden Analysenformel

$P'-(W' - A')_m$

worin
W' wie in Anspruch 2 definiert ist; m von 0,3 bis 12 variiert; P' den Rest eines Proteins P darstellt, welches ein Antikörper oder ein Antikörperfragment als solches oder zweckmäßig chemisch modifiziert ist, dem eine oder mehrere eigentliche Funktionen entzogen sind, und bei dem die anderen funktionellen Gruppen gegebenenfalls blockiert sind; A' den Rest des Proteins A darstellt, welches Trichokirin ist, wie in Anspruch 1 definiert.

**4.** Immunotoxin nach einem der Ansprüche 1 bis 3, dadurch gekennzeichnet, daß das Protein A modifiziertes Trichokirin ist, dargestellt durch die Formel

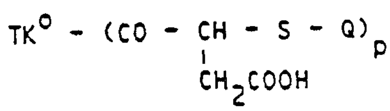

worin
$TK^o$ den Rest von Trichokirin darstellt, Q Wasserstoff oder die Gruppe Acetyl bedeutet und p zwischen 0,2 und 3 variiert.

**5.** Immunotoxin nach Anspruch 3 mit der folgenden Analysenformel

$$P'-(NH-CO-CH_2CH_2-S-S-\underset{\displaystyle CH_2COOH}{CH}-CO-NH-TK')_m$$

worin
P' und m wie in Anspruch 4 definiert sind und TK' der Rest von Trichokirin als solches ohne Aminofunktionen ist.

6. Immunotoxin nach Anspruch 3 mit der folgenden Analysenformel

$P'-(NH-CO-CH_2CH_2-S-S-CH_2CH_2-CO-O-TK'')_m$

worin
P' und m wie in Anspruch 3 definiert sind und TK" der Rest von Trichokirin als solches ohne phenolische Hydroxylfunktionen ist.

7. Verfahren zur Herstellung eines Immunotoxins mit der folgenden Analysenformel

P' - W - A'

worin
P' den Rest eines Proteins P darstellt, welches ein Antikörper oder ein Antikörperfragment als solches oder zweckmäßig chemisch modifiziert ist, dem eine oder mehrere eigentliche Funktionen entzogen sind, und bei dem die anderen funktionellen Gruppen gegebenenfalls blockiert sind; A' den Rest des Proteins A darstellt, welches Trichokirin als solches oder zweckmäßig chemisch modifiziert ist, dem eine oder mehrere eigentliche Funktionen entzogen sind, und bei dem die anderen funktionellen Gruppen gegebenenfalls blockiert sind; und W eine bivalente kovalente Struktur darstellt, die mindestens eine Thioethergruppe oder eine Disulfidgruppe enthält, bei der eines der Schwefelatome entweder ausgewählt ist aus jenen der Cysteine von P oder A oder über eine an die eigentlichen Funktionen von P und/oder von A gebundene funktionelle Gruppe tragende Raumstrukturen an die eigentlichen Funktionen von P und/oder A gebunden ist; dadurch gekennzeichnet, daß man ein Protein P1, welches ohne Unterschied entweder Trichokirin, gegebenenfalls modifiziert, oder ein Antikörper oder Antikörperfragment und Träger von mindestens einer freien Thiolgruppe ist, welche direkt oder unter Zwischenschaltung einer Raumstruktur am Protein P1 hängt, mit einem Protein P2, welches von P1 unterschiedlich und ohne Unterschied entweder Trichokirin, gegebenenfalls modifiziert, oder ein Antikörper oder Antikörperfragment und Träger einer mit dem freien Thiol des Proteins P1 kopplungsfähigen Gruppe zur Bildung einer Thioether- oder Disulfidbindung in wässeriger Lösung und bei Umgebungstemperatur zur Umsetzung bringt.

8. Verfahren nach Anspruch 7, dadurch gekennzeichnet, daß man als Protein P1 oder P2 ein Proteinderivat verwendet, dargestellt durch die Analysenformel

P2" - O - CO - E - G (VI)

worin
- P2" den Rest eines Proteins, ausgewählt aus:
  . jedem Antikörper oder Antikörperfragment oder jedem Immunglobulin oder Immunglobulinfragment oder jedem Molekül, das von den vorhergehenden durch künstliche Modifikation irgendeiner ihrer funktionellen Gruppen stammt,
  . Trichokirin oder jedem Molekül, das von diesem Protein durch künstliche Modifikation irgendeiner seiner funktionellen Gruppen stammt,

  darstellt, wobei der Rest das Protein ist, dem eine oder mehrere phenolische Hydroxylgruppen der Tyrosine entzogen sind;
- das Sauerstoffatom jenes der phenolischen Hydroxylgruppen ist, die im Rest P2" fehlen;
- E wie in Anspruch 2 definiert ist und
- G für eine Gruppe

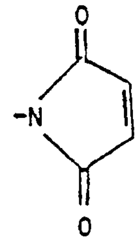

oder eine Gruppe -S-S-X, worin X eine Aktivatorgruppe ist, steht.

**9.** Verfahren nach Anspruch 7, dadurch gekennzeichnet, daß das Protein A modifiziertes Trichokirin der Formel

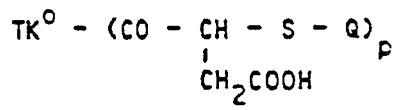

ist, worin
TK° den Rest von Trichokirin darstellt, Q für Wasserstoff oder die Gruppe Acetyl steht und p zwischen 0,2 und 3 variiert.

**10.** Verfahren nach Anspruch 7 zur Herstellung eines Immunotoxins mit der Analysenformel

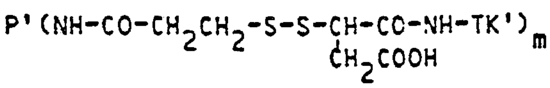 (IIIa)

worin P' und m wie in Anspruch 3 definiert sind und TK' der Rest von Trichokirin als solches ohne Aminofunktionen, ist, dadurch gekennzeichnet, daß es darin besteht, daß ein Protein P1, welches ein Antikörper oder ein Antikörperfragment und Träger von mindestens einer unter Zwischenschaltung einer Raumstruktur der Formel $-NH-CO-CH_2-CH_2-$ an das Protein P1 gebundenen freien Thiolgruppe ist, mit Mercaptosuccinoyltrichokirin als Protein P2 in wässeriger Lösung und bei Umgebungstemperatur zur Umsetzung gebracht wird.

**11.** Verfahren nach Anspruch 7 zur Herstellung eines Immunotoxins mit der Analysenformel

$P'-(NH-CO-CH_2CH_2-S-S-CH_2CH_2-CO-O-TK'')_m$ (IIIb)

worin
P' und m wie in Anspurch 3 definiert sind und TK" der Rest von Trichokirin als solches, ohne phenolische Hydroxylfunktionen ist, dadurch gekennzeichnet, daß es darin besteht, daß ein Protein P1, welches ein Antikörper oder ein Antikörperfragment und Träger von mindestens einer unter Zwischenschaltung einer Raumstruktur der Formel $-NH-CO-CH_2-CH_2-$ an das Protein P1 gebundenen freien Thiolgruppe ist, in wässeriger Lösung und bei Umgebungstemperatur mit Trichokirin-3-(2-Pyridyldisulfanyl)-propionat als Protein P2 zur Umsetzung gebracht wird.

**12.** Proteinderivate der Analysenformel

P2" - O - CO - E - G (VI)

worin
- P2" den Rest von Trichokirin oder jedem Molekül, das von diesem Protein durch künstliche Modifikation irgendeiner seiner funktionellen Gruppen stammt, darstellt;
wobei der Rest das Protein ist, dem eine oder mehrere phenolische Hydroxylgruppen der

Tyrosine entzogen sind;
- das Sauerstoffatom jenes der phenolischen Hydroxylgruppen ist, die im Rest P2" fehlen;
- E wie in Anspruch 2 definiert ist und
- G für eine Gruppe

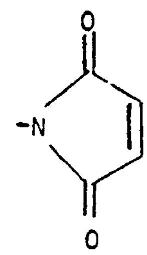

oder eine Gruppe -S-S-X, worin X eine Aktivatorgruppe ist, steht.

**13.** Verfahren zur Herstellung der Proteinderivate nach Anspruch 12, dadurch gekennzeichnet, daß es darin besteht, daß ein Produkt der Formel P2"-OH, worin P2" wie oben definiert und wobei die Hydroxylgruppe das in den Tyrosinen des Restes P2" fehlende phenolische Hydroxyl ist, mit einer Verbindung der nachstehenden Formel (VII)

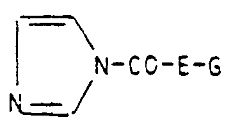

(VII)

bei einer Temperatur von 10 bis 40 °C in einem wässerigen Lösungsmittel, das gegebenenfalls ein mit Wasser mischbares organisches Lösungsmittel, wie beispielsweise ein etherisches Lösungsmittel, wie Dioxan oder Tetrahydrofuran, enthält, zur Umsetzung gebracht wird.